(19) Europäisches Patentamt
European Patent Office
Office européen des brevets

(11) Publication number: **0 105 658 A1**

(12) **EUROPEAN PATENT APPLICATION**

(21) Application number: 83305512.2

(22) Date of filing: 20.09.83

(51) Int. Cl.³: C 07 D 487/04
A 61 K 31/40
//C07F7/18, C07C149/18, C07C149/24, (C07D487/04, 209/00, 205/00)

(30) Priority: 28.09.82 GB 8227641
11.12.82 GB 8235368

(43) Date of publication of application: 18.04.84 Bulletin 84/16

(84) Designated Contracting States: BE CH DE FR GB IT LI NL SE

(71) Applicant: BEECHAM GROUP PLC
Beecham House Great West Road
Brentford Middlesex(GB)

(72) Inventor: Southgate, Robert
7 Tillets Lane
Warnham West Sussex(GB)

(72) Inventor: Corbett, David Francis
72E Holmesdale Road
Reigate Surrey(GB)

(72) Inventor: Coulton, Steven
6 Badgers Close
Horsham West Sussex(GB)

(74) Representative: Hardman, Carol Pauline et al,
Beechams Pharmaceuticals Great Burgh Yew Tree
Bottom Road
Epsom, Surrey, KT18 5XQ(GB)

(54) **Beta-lactam compounds.**

(57) An amine compound of formula (I) or an amidine derivative thereof or a pharmaceutically acceptable salt or ester thereof:

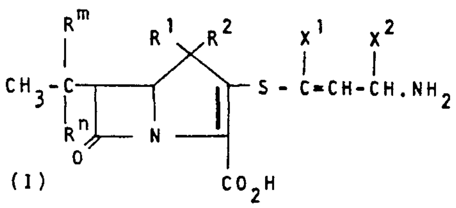

(I)

wherein $R^m$ is hydrogen or a group $-OR$, $R^n$ is hydrogen or methyl, R is hydrogen, $-CHO$ or $-SO_3H$ or a pharmaceutically acceptable salt thereof, $R^1$ and $R^2$ are independently hydrogen, $C_{1-6}$ alkyl, or $R^1$ and $R^2$ are joined so as to form with the carbon to which they are attached a $C_{3-7}$ cycloalkyl ring, and $X^1$ and $X^2$ are independently hydrogen or optionally substituted hydrocarbon; a process for the preparation of such compounds, pharmaceutical compositions comprising them and the intermediates useful in the preparation of the compounds (I).

EP 0 105 658 A1

# β-LACTAM COMPOUNDS

This invention relates to novel β-lactam containing compounds, their preparation and their use, and in particular to a novel class of carbapenems. These compounds have antibacterial properties, and therefore are of use in the treatment of bacterial infections in humans and animals caused by a wide range of organisms.

The present invention relates to an amine compound of formula (I) or an amidine derivative thereof or a pharmaceutically acceptable salt or ester thereof:

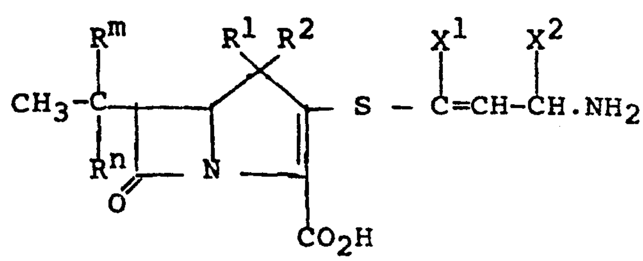

(I)

wherein $R^m$ is hydrogen or a group -OR, $R^n$ is hydrogen or methyl, R is hydrogen, -CHO or $-SO_3H$ or a pharmaceutically acceptable salt thereof, $R^1$ and $R^2$ are independently hydrogen, $C_{1-6}$ alkyl, or $R^1$ and $R^2$ are joined so as to form with the carbon to which they are attached a $C_{3-7}$ cycloalkyl ring, and $X^1$ and $X^2$ are independently hydrogen or optionally substituted hydrocarbon.

Suitably R is $-SO_3H$ or a pharmaceutically acceptable salt thereof such as an alkali metal salt, for example the sodium or potassium salt.

Preferably $R^m$ represents a group -OR, and $R^n$ represents hydrogen.

Preferably R is hydrogen or -CHO.

In particular, the amidine derivatives of this invention include compounds of formula (II) or a pharmaceutically acceptable salt or ester thereof:

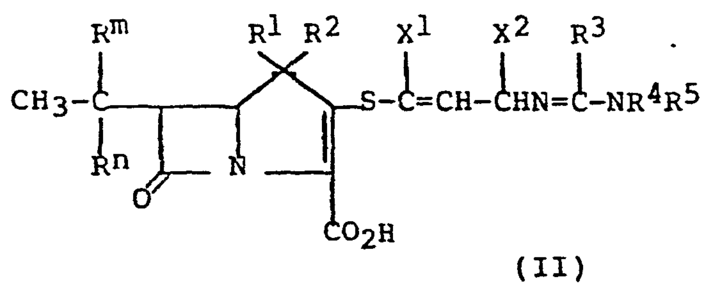

(II)

wherein $R^m$, $R^n$, $R^1$, $R^2$, $X^1$ and $X^2$ are as defined with respect to formula (I) and $R^3$, $R^4$ and $R^5$ are independently selected from hydrogen or an optionally substituted hydrocarbon or heterocyclic group; or $R^4$ and $R^5$ together with the nitrogen to which they are attached form a 3-10 membered heterocyclic ring.

Suitably $R^3$, $R^4$ and $R^5$ are independently hydrogen or $C_{1-6}$ alkyl.

Preferably $R^3$, $R^4$ and $R^5$ are independently hydrogen or methyl.

Preferably $X^1$ and $X^2$ independently represent hydrogen, $C_{1-6}$ alkyl or optionally substituted aryl.

In particular $X^1$ and $X^2$ independently represent hydrogen, methyl or phenyl.

The compounds wherein R is -CHO are active in their own right but it is possible that they could be converted to compounds wherein R is hydrogen in-vivo.

Suitably $R^1$ is hydrogen, methyl or ethyl. Suitably $R^2$ is hydrogen, methyl or ethyl. Preferably $R^1$ and $R^2$ are both hydrogen.

Examples of suitable pharmaceutically acceptable in vivo hydrolysable ester groups include those which break down readily in the human body to leave the parent acid or its salts, for example acyloxyalkyl groups, such as acetoxymethyl, pivaloyloxymethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl groups, such as dimethylaminomethyl, dimethylaminoethyl, diethylaminomethyl or diethylaminoethyl; and lactone groups such as phthalidyl or dimethoxyphthalidyl.

Suitable pharmaceutically acceptable salts of the compounds of formula (I) include metal salts, eg

aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabrietylamine, N,N'-bisdehydroabietylamine, ethylenediamine, or bases of the pyridine type such as pyridine, collidine or quinoline.

As used herein the term 'hydrocarbon' includes groups having up to 18 carbon atoms, suitably up to 10 carbon atoms, conveniently up to 6 carbon atoms. Suitable hydrocarbon groups include $C_{1-6}$ alkyl, $C_{2-6}$ alkenyl, $C_{2-6}$ alkynyl, $C_{3-7}$ cycloalkyl, $C_{3-7}$ cycloalkyl($C_{1-6}$)-alkyl, aryl, and aryl($C_{1-6}$)alkyl.

Suitable alkyl groups include straight and branched chain alkyl groups containing from 1 to 6 carbon atoms, such as methyl, ethyl, propyl and butyl. A particular alkyl group is methyl.

The term 'heterocyclyl' includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-($C_{1-6}$)-alkyl, hydroxy, amino, carboxy, $C_{1-6}$ alkoxycarbonyl, $C_{1-6}$ alkoxycarbonyl($C_{1-6}$) alkyl, aryl or oxo groups.

Suitably the heterocyclic ring comprises from 4 to 7 ring atoms, preferably 5 to 6 atoms.

When used herein the term 'aryl' includes phenyl and naphthyl optionally substituted with up to five,

preferably up to three, groups selected from halogen, $C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl $C_{1-6}$ alkylcarbonyloxy, or $C_{1-6}$ alkylcarbonyl groups.

Suitable optional substituents for the hydrocarbon, heterocyclic groups and organic radicals include $C_{1-6}$ alkyl, heterocyclic, amino, $C_{1-6}$ alkanoyl-amino, mono, di- and tri- ($C_{1-6}$) alkylamino, hydroxy, $C_{1-6}$ alkoxy, mercapto, $C_{1-6}$ alkylthio, heterocyclyl-thio, arylthio, sulphamoyl, carbamoyl, amidino, guanidino, nitro, chloro, bromo, fluoro, carboxy and salts and esters thereof, $C_{1-6}$ alkanoyloxy, aryl-carbonyl and heterocyclylcarbonyl.

The compounds of this invention may have R or S stereochemistry at the C-8 position (that is the $\alpha$-carbon atom of the C-6 substituent) or may be in the form of mixtures thereof.

The compounds of this invention may be provided with a cis configuration of the C-5 and C-6 protons, or they may be provided with a trans configuration of the C-5 and C-6 protons. Alternatively they are provided as a mixture of the cis and trans forms.

Preferred configurations for the compounds of this invention with an asymmetric 8-position are 5R, 6R, 8S, and 5R, 6S, 8R.

The double bond present in the C-3 thio substituent may be present in either the E configuration or the Z configuration, or the compounds may be provided as mixtures thereof.

Preferably the double bond is in the Z (or cis) configuration.

The compounds most suitably may be presented in the form of the carboxylic free acid at the C-2 position, said compounds preferably being in zwitterion form.

Preferred compounds of this invention are compounds of formula (I), wherein $R^m$ is OH, and $R^n$, $R^1$, and $R^2$ are hydrogen:

(5R,6R)-3-[(Z)-3-aminoprop-1-enylthio]-6-[(S)-1-hydroxyethyl]-7-oxo-1-azabicyclo-[3.2.0]hept-2-ene -2-carboxylic acid;

(5R,6S)-3-[(Z)-3-aminoprop-1-enylthio]-6-[(R)-1-hydroxyethyl]-7-oxo-1-azabicyclo[3.2.0]hept-2-ene-2-carboxylic acid;

(5R,6R)-3-(3-aminobut-1-en-1-ylthio)-6-[(S)-1-hydroxyethyl]-7-oxo-1-azabicyclo[3.2.0]hept-2-ene-2-carboxylic acid;

(5R,6S)-3-(3-aminobut-1-en-1-ylthio)-6-[(R)-1-formyloxyethyl]-7-oxo-1-azabicyclo[3.2.0]hept-2-ene-2-carboxylic acid;

(5R,6R)-3-[(Z)-3-acetimidoylaminoprop-1-enylthio]-6-[(S)-1-hydroxyethyl]-7-oxo-1-azabicyclo[3.2.0]hept-2-ene-2-carboxylic acid;

(5R,6R)-3-[(Z)-3-formimidoyl-aminoprop-1-enylthio] 6-[(S)-1-hydroxyethyl]-7-oxo-1-azabicyclo[3.2.0]hept-2-ene-2-carboxylic acid;

(5R,6S)-3-(3-aminobut-1-en-1-ylthio)-6-[(R)-1-hydroxyethyl]-7-oxo-1-azabicyclo[3.2.0]hept-2-ene-2-carboxylic acid;

(5R,6R)-3-[(Z)-3-amino-1-methylprop-1-enylthio]-6-[(S)-1-hydroxyethyl]-7-oxo-1-azabicyclo[3.2.0]hept-2-ene-2-carboxylic acid;

(5R,6R)-3-(3-amino-1-phenylprop-1-enylthio)-6-[(S)-1-hydroxyethyl]-7-oxo-1-azabicyclo[3.2.0]hept-2-ene-2-carboxylic acid;

(5R,6S)-3-[(Z)-3-amino-1-methylprop-1-enylthio]-6-[(R)-1-hydroxyethyl]-7-oxo-1-azabicyclo[3.2.0]hept-2-ene-2-carboxylic acid;

(5R,6R)-3-[(E)-3-aminoprop-1-enylthio]-6-[(S)-1-hydroxyethyl]-7-oxo-1-azabicyclo[3.2.0]hept-2-ene-2-carboxylic acid;

(5R,6S)-3-[(E)-3-aminoprop-1-enylthio]-6-[(R)-1-hydroxyethyl]-7-oxo-1-azabicyclo[3.2.0]hept-2-ene-2-carboxylic acid;

(5RS,6SR)-3-[(Z)-3-aminoprop-1-enylthio]-6-[(1RS)-1-hydroxyethyl]-7-oxo-1-azabicyclo[3.2.0]hept-2-ene-2-carboxylic acid;

(5RS,6SR)-3-[(E)-3-amino-1-methylprop-1-enylthio]-6-[(RS)-1-hydroxyethyl]-7-oxo-1-azabicyclo[3.2.0]hept-2-ene-2-carboxylic acid;

(5R,6R)-3-[(E)-3-amino-1-methylprop-1-enylthio]-6-[(S)-1-hydroxyethyl]-7-oxo-1-azabicyclo[3.2.0]hept-2-ene-2-carboxylic acid.

The compounds of this invention may be prepared by a process which comprises reacting a compound of formula (III):

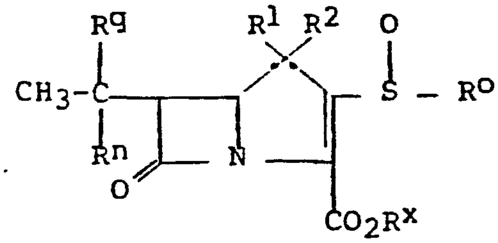

(III)

wherein $R^1$ and $R^2$ are as defined with respect to formula (I), $R^q$ is hydrogen or a group $-OR^p$, where $R^p$ is a group R as defined with respect to formula (I) or a protected derivative thereof, $R^x$ is a carboxy protecting group and $R^o$ is an optionally substituted hydrocarbon or heterocyclic group;

with a compound of formula (IV) or a salt or an amidine derivative thereof:

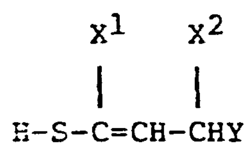

(IV)

where Y is amino, protected amino, or a precursor thereof,and optionally thereafter removing any protecting groups and converting the product into a pharmaceutically acceptable salt or ester.

Compounds of formula (III) may be prepared as described in European Patent Application Publication Numbers 0,060,077 and 0,060,612.

Suitable protected derivatives $R^p$ of the group R are those conventional in the art, for example groups cleavable by hydrolysis such as formate or silyl groups or groups cleavable by hydrogenation such as benzyloxycarbonyloxy and p-nitrobenzyloxycarbonyloxy.

Suitably Y is amino or protected amino.

Suitable amino-protecting groups are those well-known in the art which may be removed under conventional conditions without disruption of the remainder of the molecule.

Examples of N-protecting groups include $C_{1-6}$ alkanoyl, for example acetyl, propionyl n- and isobutyryl and 2,2-dimethylpropanoyl, benzoyl or benzene optionally substituted in the phenyl ring by one or two substituents selected from $C_{1-4}$ alkyl, $C_{1-4}$ alkoxy, trifluoromethyl, halogen or nitro; and $C_{1-4}$ alkoxycarbonyl, for example tert-butoxycarbonyl; or benzyl optionally substituted as for benzoyl above.

Alternatively the group Y may be a precursor of an amino groups, such as hydroxy or azido.

This reaction may be performed in any solvent that is substantially inert during the reaction, for example tetrahydrofuran, dimethylformamide, dioxan, hexamethyl phosphoramide, dimethoxyethane or dimethoxydiethyl ether. Of these solvents dimethylformamide is preferred. Alternatively we have found it useful to use a phase transfer catalysts such as tetra-n-butyl ammonium bromide, cetyl benzyl dimethylammonium chloride and cetyltriethylammonium chloride; suitable solvents include halogenated water-immiscible solvents such as chloroform in the presence of water. The reaction is normally performed at ambient or a depressed temperature, for example 20°C to -70°C, and preferably between 0°C and -50°C. However, when using a phase transfer catalyst it is preferable to conduct the reaction between 0°C and ambient temperature. When the thiol of the formula (IV) is used the reaction is normally carried out in the presence of a base.

Examples of such bases include sodium hydride, sodium hydroxide, sodium alkoxide such as the methoxide, ethoxide or butoxide, sodium amide, potassium hydroxide, potassium alkoxide such as the methoxide ethoxide or butoxide, potassium amide, and trialkylamines such as triethylamine and tri-n-propylamine.

Of these triethylamine is preferred. Preferably the base is present in an amount of at least 0.9 equivalents, more preferably between 1.0 and 1.2 equivalents per mole of the thiol compound. Instead of using a base in the reaction a reactive derivative of the thiol may be used, preferably the reactive derivative is a salt of the thiol, in particular an alkali metal salt such as sodium or potassium. The amount of thiol compound of the formula (IV) or reactive derivative is generally between 1.0 and 1.5 moles per mole equivalent of the compound of the formula (III).

Suitable carboxyl-protecting derivatives for the group $-CO_2R^x$ in formula (III) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2,6-lutidine, pyridine, N-methylpyrrolidine, dimethylpiperazine. A preferred salt is with triethylamine.

Suitable ester-forming carboxyl-protecting groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, allyl, acetonyl, t-butyl, t-amyl.

diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, an oxime radical of formula $-N=CHR^o$ where $R^o$ is aryl or heterocyclic, or an in vivo hydrolysable ester radical.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

The compounds of this invention may also be prepared by a process which comprises the elimination of the elements of $R^aR^bR^cP=O$ from an ester of a compound of the formula (V) or an amidine derivative thereof:

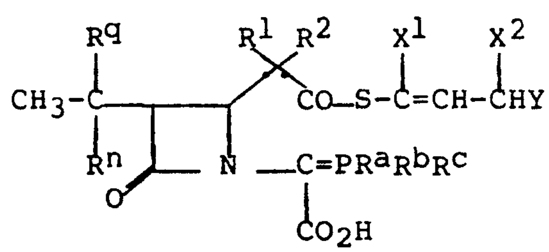

(V)

wherein $R^q$, $R^n$, $X^1$, $X^2$, $R^1$, $R^2$ and Y are as hereinbefore defined and $R^a$, $R^b$ and $R^c$ are independently phenyl or methoxyphenyl and optionally thereafter removing any protecting and/or blocking groups and converting the product into a pharmaceutically acceptable salt or ester.

Suitably this process is performed by heating the ester of the compound of the formula (V) in an inert solvent, for example at temperatures of 90°C to 120°C and more suitably 100°C to 110°C in a solvent such as toluene, preferably under substantially anhydrous conditions.

The preparation of the compounds of the formula (V) may be effected by the reaction of a compound of the formula (VI):

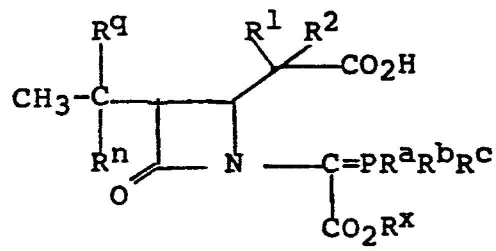

(VI)

wherein $R^q$, $R^n$, $R^1$, $R^2$, $R^x$, $R^a$, $R^b$ and $R^c$ are as hereinbefore defined; with a di($C_{1-6}$)alkylphosphorochloridate or thionyl chloride and a tri($C_{1-6}$)alkylamine followed by reaction with a derivative of the formula (VII):

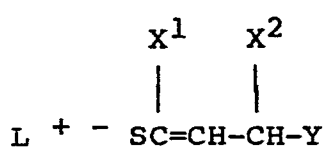

(VII)

or an amidine derivative thereof, wherein $X^1$, $X^2$ and Y are as hereinbefore defined and $L^+$ is a lithium, sodium, silver or thallium (I) cation or an ammonium ion substituted by up to three $C_{1-6}$ alkyl groups.

When $L^+$ is a substituted ammonium ion, it is preferably a tertiary ammonium ion, such as the triethylammonium ion.

Preferably $L^+$ is a thallium (I) cation or a silver cation.

A particularly suitable di-loweralkylphosphorochloride is diethylphosphorochloridate. A particularly suitable tri-loweralkylamine is triethylamine.

The reaction is generally carried out in an inert organic solvent such as tetrahydrofuran at a non-extreme temperature such as 0°C to 40°C, for example 15°C to 25°C.

The compounds of the formula (VI) may be prepared by the methods of European Patent Application Publication Numbers: 0008888, 0008514, 0002564 and 0060612.

The amine compounds of formula (I) may be prepared by a process which comprises the reduction of a compound of formula (X):

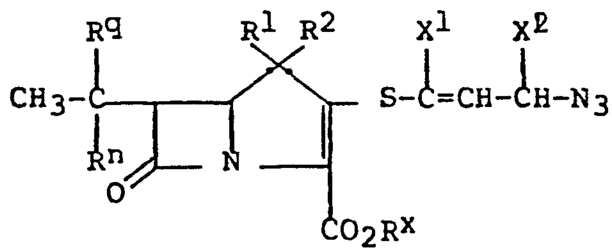

(X)

wherein $R^1$, $R^2$, $R^q$, $R^n$ and $R^x$ are as hereinbefore defined

The reduction of compound (X) may be carried out by any conventional reduction methods such as by hydrogenation or the use of other reducing agents such as hydrogen sulphide/triethylamine.

The hydrogenation is normally carried out in the presence of a transition metal catalyst, such as palladium, for example in the form of palladium on carbon (charcoal), palladium on barium sulphate, palladium on calcium carbonate, and palladium black. A favoured catalyst is palladium on carbon (sometimes referred to as palladium on charcoal); for example 5%, 10%, 20% or 30% palladium on carbon. A low, medium or high pressure of hydrogen may be used in this reaction, for example from 1 to 6 atmospheres. The reaction is normally carried out at a non-extreme temperature, for example from 0°C to 30°C and more usually from 12°C to 25°C. It is generally convenient to carry out the reaction at ambient temperature. Suitable solvents for carrying out the hydrogenation include ethanol, n-propanol, isopropanol, tetrahydrofuran, dioxan, ethyl acetate or mixtures of such solvents or such solvents in the presence of water. A favoured solvent is aqueous 1,4-dioxan, preferaly buffered to neutral pH.

The compound of formula (X) may be prepared by reacting a compound of formula (XI):

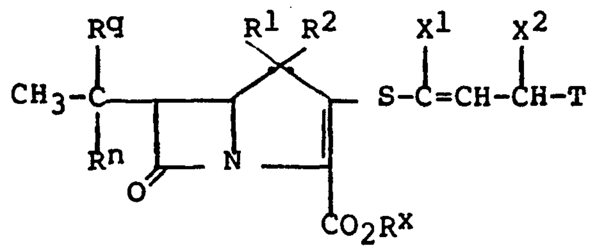

(XI)

wherein $R^1$, $R^2$, $R^q$, $R^n$ and $R^x$ are as hereinbefore defined and T represents a readily displaceable group; with an azide.

Suitably the azide is an inorganic azide such as an alkali metal azide e.g. sodium azide or lithium azide; or an organic azide such as tetramethylguanidium azide, or tetrabutylammonium azide.

The readily displaceable group T may be for example a halogen atom (e.g. chlorine, bromine or iodine) or an acyloxy group such as an optionally substituted hydrocarbon-carbonyloxy or sulphonyloxy group. Suitable such groups include dichloroacetoxy, methanesulphonyloxy, fluoromethanesulphonyloxy, p-toluenesulphonyloxy, p-nitrobenenesulphonyloxy or tri-fluoromethylsulphonyloxy.

Compounds of formula (XI) wherein T is an acyloxy group may be prepared by reacting a hydroxy compound of formula (XII) with an appropriate acylating agent e.g. an acyl halide or anhydride.

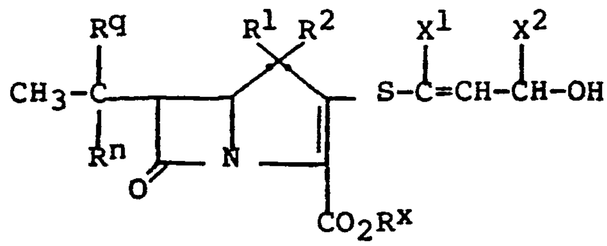

(XII)

Compounds of formula (XI) wherein T is halogen may be obtained by reaction of compound (XII) with a halogenating agent such as a thionyl halide.

In a preferred method of preparing the compound of formula (X), the intermediate activated derivative (XI) is not isolated. In this aspect, the hydroxy compound (XII) is reacted with

i) a compound of formula (XIII):

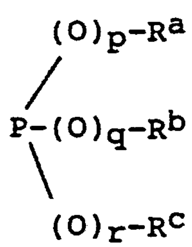

wherein p, q and r are independently zero or 1 and $R^a$, $R^b$ and $R^c$ are each independently $C_{1-6}$ alkyl, aryl or aryl($C_{1-6}$)alkyl;

ii) hydrazoic acid; and

iii) a compound of formula (XIV):

$R^dO.CON{=}NCO.OR^e$

wherein $R^d$ and $R^e$ are independently $C_{1-6}$ alkyl, aryl or aryl($C_{1-6}$)alkyl.

Suitable compounds of the formula (XIII) include those wherein the $R^a$, $R^b$ and $R^c$ groups are selected from methyl, ethyl, n-propyl, n-butyl, benzyl, phenyl and methoxyphenyl groups. It is generally convenient that $R^a$, $R^b$ and $R^c$ each represent the same moiety. Favoured compounds of the formula (XIII) include tri-aryl-phosphines and tri-alkylphosphites. Particularly suitable compounds of the formula (XIII) include triphenylphosphine, trimethylphosphite, tri-ethyl-phosphite, tri-p-methoxyphenylphosphine and tri-n-butylphosphine.

Suitable compounds of the formula (XIV) include those wherein $R^d$ and $R^e$ are independently selected from methyl, ethyl, propyl, butyl, phenyl and benzyl groups. It is generally convenient that $R^d$ and $R^e$ represent the same moiety. Particularly suitable compounds of the formula (XIV) include those wherein $R^d$ and $R^e$ each represent a methyl, ethyl, t-butyl or isopropyl group.

Particular compounds of formula (XIV) include diethylazodicarboxylate and diisopropylazodicarboxylate.

The reaction is performed in an inert organic solvent. The solvent used should be aprotic and substantially unreactive towards the reagents involved. Suitable solvents include tetrahydrofuran, dioxan, ethyl acetate, benzene, toluene and chlorobenzene. Of these tetrahydrofuran is preferred.

The reaction is normally carried out at a non-extreme temperature such as -20°C to +100°C, more usually from about 5°C to 50°C and conveniently at ambient temperature (approximately 15°C to 25°C).

The hydroxy compound of formula (XII) may be prepared by reduction of a compound of formula (XV):

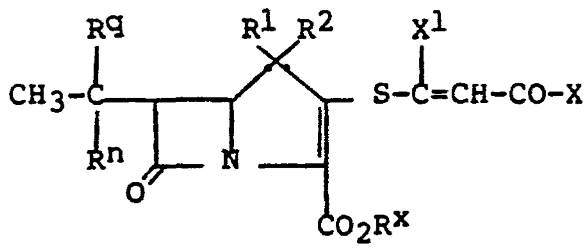

, (XV)

wherein X represents $X^2$ or $OR^y$ where $CO_2R^y$ represents an ester group. Suitably $R^y$ is a hydrocarbon radical, such as $C_{1-6}$ alkyl, preferably methyl or ethyl.

A suitable method of reduction utilises a complex hydride such as a borohydride, for example lithium borohydride, sodium or potassium borohydride, sodium cyanoborohydride, di-isobutyl aluminium hydride; or a reagent such as aluminium tri-isopropoxide. The preferred reducing agent is selected according to the nature of the compound (XV), i.e. whether it is an aldehyde, ketone or ester. The reaction may be performed in an inert organic solvent such as a hydrocarbon, a chlorinated hydrocarbon or an ether, for example toluene, cyclohexane, diethyl ether, heptane, hexane, dichloromethane or tetrahydrofuran. The

reaction may be performed at any non-extreme temperature for example -30°C to +60°C, more suitably -20°C to 0°C.

Compounds of formula (XV) are prepared as disclosed in European Patent Application Publication No 0,024,832.

Compounds of formulae (X), (XI), (XII) and compounds (XV) where X represents $X^2$ are novel compounds and form a further aspect of this invention.

The amidine compounds of formula (II) may be prepared by a process which comprises reaction of a compound of formula (I) or a protected derivative thereof with either a compound of formula (VIII) or (IX):

$$\begin{array}{c} R^3 \\ | \\ R^6\text{-}Y\text{-}C\text{=}NR^4 \end{array} \qquad (VIII)$$

$$\begin{array}{c} R^3 \\ | \\ X\text{-}C\text{=}^{+}NR^4R^5 \end{array} \quad Z^- \qquad (IX)$$

wherein $R^3$, $R^4$ and $R^5$ are as defined with respect to formula (II), $R^6$ is $C_{1-6}$ alkyl, Y is oxygen or sulphur, X is halogen or $C_{1-6}$ alkoxy and Z is a salting ion.

The compound of the formula (VIII) is preferably used in the form of its acid addition salt, for example as the hydrochloride.

Suitably $R^6$ is a methyl or ethyl group. Suitably Y is an oxygen atom. Suitably also X is C1-6 alkoxy

such as methoxy or ethoxy. Suitably Z is halo such as chloro, or is a tetrafluoroborate ion.

Reaction of the compound of the formula (I) with either the compound of the formula (VIII) or (IX) is suitably performed in water, ether, dioxan, tetra-hydrofuran, dimethylformamide, dichloromethane or mixtures of such solvents. Suitably the reaction isperformed between 0°C and ambient temperature. In aqueous media it has been found convenient to carry out the reaction at a basic pH, for example 7.5 to 10, preferably 8 to 9.

The present invention also provides a pharmaceutical composition which comprises a compound of this invention and a pharmaceutically acceptable carrier.

The compositions of the invention include those in a form adapted for oral, topical or parenteral use and may be used for the treatment of the infection in animals especially mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

The injectable solution of the compound of this invention may be made up in a sterile pyrogen-free liquid such as water, aqueous ethanol or the like.

Unit dose compositions comprising a compound of this invention adapted for oral administration form a further suitable composition aspect of this invention.

The compositions may contain from 0.1% by weight, preferably from 10-60% by weight, of the active material, depending on the method of administration. Where the composition comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

The compound of the formula may be present in the composition as sole therapeutic agent or it may be present together with other therapeutic agents such as, for example, a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other well known penicillins including pro-drugs thereof such as their in vivo

hydrolysble esters such as the acetoxymethyl, pivaloyloxymethyl, α-ethoxycarbonyloxyethyl or phthalidyl esters of ampicillin benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include, for example, cefatrizine, cephaloridine, cephalothin, cefazolin, cephalexin, cephacetrile, cephapirin, cephamandole nafate, cephradine, 4-hydroxycephalexin, cefaparole, cephaloglycin, cefoperazone, and other well known cephalosporins or pro-drugs thereof.

When present together with a cephalosporin or penicillin, the ratio of a compound of the invention to the penicillin or cephalosporin agent may vary over a wide range of ratios, such as from 10:1 to 1:10, for example about 3:1, 2:1, 1:1, 1:2, 1:3, 1:4, 1:5 or 1:6 (wt/wt, based on pure free antibiotic equivalent).

The antibiotic compounds of the present invention are active against a wide range of gram negative and gram positive organisms.

The MIC data included in the following examples is representative of the activity of the compounds of the present invention.

The following Examples illustrate this invention.

Preparation 1

p-Nitrobenzyl(5R,6R)-3-[(Z)-2-ethoxycarbonylethenylthio]-6-[(S)-1-trimethylsilyloxyethyl]-7-oxo-1-azabicyclo[3.2.0]hept-2-ene-2-carboxylate

p-Nitrobenzyl (5R,6R)-3-[(Z)-2-ethoxycarbonylethenylthio]-6-[(S)-1-hydroxyethyl]-7-oxo-1-azabicyclo [3.2.0]hept-2-ene-2-carboxylate (0.757 gm) (el) was dissolved in dry pyridine (5 ml) and stirred at room temperature for 30 minutes with trimethylsilyl chloride (1.068 gm). The solvent was then evaporated at reduced pressure and the residue was partitioned between ethyl acetate and water. The organic layer was washed with saturated sodium chloride solution, dried over anhydrous magnesium sulphate and evaporated at reduced pressure. The crude product was chromatographed over silica gel (30 gm). Elution with 75% ethyl acetate/hexane afforded the title compound as a pale yellow oil, which crystallised on standing (0.666 gm). $\gamma$ max ($CHCl_3$) 1782, 1708, 1608, 1580, 1559, 1521, 842 $cm^{-1}$.

Preparation 2

p-Nitrobenzyl(5R,6R)-3-[(Z)-3-hydroxyprop-1-enylthio]-6-[(S)-1-trimethylsilyloxyethyl]-7-oxo-1-azabicyclo-[3.2.0]hept-2-ene-2-carboxylate

The ester p-Nitrobenzyl(5R,6R)-3-[(Z)-2 -ethoxycarbonylethenylthio]-6-[(S)-1-trimethylsilyloxyethyl]-7-oxo-1-aza-bicyclo[3.2.0]hept-2-ene-2-carboxylate (0.360 gm) was dissolved in dry tetrahydrofuran (30 ml) and cooled to $0^{o}C$ under an atmosphere of argon, with stirring. A solution of diisobutylaluminium hydride in toluene (25% w/v; 1.15 ml, 2.02 mH) was added and stirring was continued for 1.5 hours. The reaction was quenched by the addition of aqueous ethanol and the solution partitioned between ethyl acetate and water and filtered. The organic layer was washed with water,

saturated sodium chloride solution, dried over magnesium sulphate and evaporated at reduced pressure. The residue was chromatographed over silica gel. Elution with 50% ethyl acetate/hexane afforded initially unreacted starting material (0.040 gm). Continued elution with 50% ethyl acetate/hexane gave the title compound (0.100 gm), $\lambda$ max (EtOH) 323, 264 nm, $\nu$max ($CHCl_3$) 3600, 1782, 1701, 1609, 1559, 1521, 841 $cm^{-1}$.

Preparation 3

p-Nitrobenzyl(5R,6R)-3-[(Z)-3-azidoprop-1-enylthio]-6-[(S)-1-trimethylsilyloxyethyl]-7-oxo-1-azabicyclo-[3.2.0]hept-2-ene-2-carboxylate

p-Nitrobenzyl(5R,6R)-3-[(Z)-3-hydroxyprop-1-enylthio]-6-[(S)-1-trimethylsilyloxyethyl]-7-oxo-1-aza-bicyclo[3.2.0]hept-2-ene-2-carboxylate (0.078 gm) was dissolved in dry tetrahydrofuran (25 ml) and cooled to $0^{o}C$. Triphenylphosphine (0.137 gm), hydrazoic acid (1.70 M solution in toluene; 0.3 ml) and diisopropyl-azodicarboxylate (0.106 gm) were added to the solution, which was stirred at room temperature for 2.25 hours. The solution was partitioned between ethyl acetate and water. The organic layer was washed with sodium bicarbonate solution, saturated sodium chloride solution, dried over anhydrous magnesium sulphate and evaporated at reduced pressure. The crude product was chromatographed over silica gel (20 gm). Elution with 40% ethyl acetate/hexane afforded the title compound as a pale yellow oil (0.041 gm), $\lambda$ max (EtOH) 322 nm, 263 nm, $\nu$ max ($CHCl_3$) 2100, 1783, 1704, 1562, 1521, 842 $cm^{-1}$, $_H$($CDCl_3$) 1.22(3H, d, $C\underline{H}_3CH$), 2.93 (1H, dd, 4-Ha), 3.4-4.0 (4H, m, 4-Hb + $CH_2N_3$), 4.05-4.4 (2H, m, 5-H +8-H), 5.28 (2H, q, $CH_2Ar$), 5.89 (1H, dt, $C{=}C\underline{H}CH_2N_3$), 6.45(1H, d, $SC\underline{H}{=}C$), 7.55(2H, d, aromatic), 8.11(2H, d, aromatic).

Example 1

(5R,6R)-3-[(Z)-3-aminoprop-1-enylthio]-6-[(S)-1-hydroxyethyl]-7-oxo-1-azabicyclo[3.2.0]hept-2-ene-2-carboxylic acid

p-Nitrobenzyl(5R,6R)-3-[(Z)-3-azidoprop-1-enylthio]-6-[(S)-1-trimethylsilyloxyethyl]-7-oxo-1-azabicyclo-[3.2.0]hept-2-ene-2-carboxylate (20 mg) was dissolved in 1,4-dioxan (9 ml), water (2 ml) and pH 7.0 0.05M phosphate buffer (3 ml) and shaken at ambient temperature and pressure for 1 hour 25 minutes in the presence of hydrogen and 5% palladium on carbon catalyst (30 mg). The suspension was filtered over Celite, washing well with water (20 ml). The filtrate was concentrated to approximately 20 ml by evaporation at reduced pressure and washed with ethyl acetate (3 x 50 ml). The pH of the aqueous solution was adjusted to 2.5 by the addition of dilute hydrochloric acid and immediately neutralised by the addition of dilute sodium hydroxide solution. The aqueous solution was concentrated to small volume at reduced pressure and chromatographed over HP-20. Elution with water afforded an aqueous solution containing the title compound (estimated to contain 1.1 mg based on $\varepsilon$15000 at $\lambda$max 301 nm in ultra violet spectrum).

MIC against E. coli JT39 : 0.4 µg/ml
Pseudomonas aeruginosa A : 6.2 µg/ml.

## Preparation 4

## p-Nitrobenzyl (5R,6S)-3-[(Z)-2-methoxycarbonylethenylthio]-6-[(R)-1-trimethylsilyloxyethyl]-7-oxo-1-azabicyclo[3.2.0]hept-2-ene-2-carboxylate

The title compound was prepared in quantitative yield by reaction of p-Nitrobenzyl (5R,6S)-3-[(Z)-2-methoxycarbonylethenylthio]-6-[(R)-1-hydroxyethyl]-7-oxo-1-azabicyclo[3.2.0]hept-2-ene-2-carboxylate with trimethylsilyl chloride in pyridine by the procedure outlined in preparation 1, $\nu$ max ($CHCl_3$) 1780, 1705, 1602, 1590, 1550, 1520 and 841 $cm^{-1}$.

## Preparation 5

## p-Nitrobenzyl (5R,6S)-3-[(Z)-3-hydroxyprop-1-enylthio]-6-[(R)-1-trimethylsilyloxyethyl]-7-oxo-1-azabicyclo-[3.2.0]hept-2-ene-2-carboxylate

The title compound was prepared in 8% yield by diisobutyl aluminium hydride reduction of p-nitrobenzyl (5R,6S)-3-[(Z)-2-methoxycarbonylethenylthio]-6-[(R)-1-trimethylsilyoxyethyl]-7-oxo-1-azabicyclo[3.2.0]hept-2-ene-2-carboxylate, following the procedure outline in preparation 2, $\nu$ max ($CHCl_3$) 1778, 1700, 1603, 1550, 1520 and 842, $\lambda$ max ($CH_3CN$) 324 nm, 266 nm, $\delta_H$($CDCl_3$), 0.15 (9H, s, $SiMe_3$), 1.28 (3H, d, MeCH), 3.0-3.17(1H, dd, J 8Hz and 18Hz, 4-Ha), 3.18 (1H, dd, J 3Hz and 6.5Hz, 6-H), 3.24-3.37 (1H, dd, J 10Hz and 18Hz, 4-$H_b$), 4.2-4.28 (2H, m, 5-H + 8-H), 4.37 (2H, d, J 6$H_4$, $CH_2OH$), 5.39 (2H, ABq, $CH_2Ar$), 6.09 (1H, dt, J 6Hz and 10.0Hz, SCH=CH), 6.34(1H, dt, J 10Hz and ca. 1Hz), 7.66 (2H, d, aromatic), 8.22(2H, d, aromatic).

Preparation 6

p-Nitrobenzyl (5R,6S)-3-[(Z)-3-azidoprop-1-enylthio]-6-[(R)-1-trimethylsilyloxyethyl]-7-oxo-1-azabicyclo-[3.2.0]hept-2-ene-2-carboxylate

Reaction of p-nitrobenzyl (5R,6S)-3-[(Z)-3-hydroxyprop-1-enylthio]-6-[(R)-1-trimethylsilyloxy-ethyl]-7-oxo-1-azabicyclo[3.2.0]hept-2-ene-2-carboxylate with triphenylphosphine, diisopropyl-azodicarboxylate and hydrazoic acid, as described in preparation 3, afforded the title compound in 56% yield, $\nu$max ($CHCl_3$) 2100, 1780, 1700, 1603, 1559, 1520 and 841 $cm^{-1}$, $\lambda$max ($CH_3CN$) 322,264 nm, $_H$($CDCl_3$), 0.15(9H, s, Si$Me_3$), 1.28 (3H, d, MeCH), 3.02-3.16 (1H, dd, J 8.3Hz and 18.0$H_z$, 4-$H_a$), 3.20 (1H, dd, J 2.8 and 6.5Hz, 6-H), 3.23-3.40 (1H, dd, J 10.0 and 18.0Hz, 4-$H_b$), 4.03 (2H, d, $CH_2N_3$), 4.13-4.30 (2H, m, 5-H + 8H), 5.41 (2H, ABq, $CH_2$Ar), 5.98 (1H, dt, J 6.5Hz and 9.5Hz, SCH=CH), 6.54 (1H, dt, J 1Hz and 9.5Hz, SCH=CH), 7.67 (2H, d, aromatics), 8.22 (2H, d, aromatics).

Example 2

(5R,6S)-3-[(Z)-3-aminoprop-1-enylthio]-6-[(R)-1-hydroxy ethyl]-7-oxo-1-azabicyclo[3.2.0]hept-2-ene-2-carboxylic acid

Hydrogenolysis and hydrolysis of p-nitrobenzyl-(5R,6S)-3-[(Z)-3-azidoprop-1-enylthio]-6-[(R)-1-trimethylsilyloxyethyl]-7-oxo-1-azabicyclo[3.2.0]-hept-2-ene-2-carboxylate by the procedure described in Example 1 afforded an aqueous solution containing the title compound, $\lambda$max ($H_2O$) 301 nm.

Preparation 7

p-Nitrobenzyl(5R,6R)-3-[(Z)-3-oxobut-1-en-1ylthio]-6-[(S)-1-hydroxyethyl]-7-oxo-1-azabicyclo[3.2.0]hept-2-ene-2-carboxylate and the 3-[(E)]isomer

A solution of p-nitrobenzyl (5R,6R)-3-[(E)-2-acetamidoethenylthio]-6-[(S)-1-hydroxyethyl]-7-oxo-1-azabicyclo[3.2.0]hept-2-ene-2-carboxylate (200 mg) in 15% aqueous dioxan (11.5 ml) was stirred with N-bromoacetamide (62 mg) for 5 min. Chloroform was added and the organic solution was washed with water and dilute brine. The solution was dried ($MgSO_4$) and evaporated in vacuo to afford a foam which was dissolved in DMF (5 ml). To the solution was added $K_2CO_3$ (62 mg) and but-3-yn-2-one (61 mg) and the mixture was stirred for 25 min. Ethyl acetate was added and the solution was washed with water (x 5) and brine. Evaporation of the dried ($mgSO_4$) organic layer gave a crude product which was chromatographed on silica-gel employing a gradient elution of 30% hexane-ethyl acetate to 100% ethyl acetate. First-eluted was the (E)-isomer of the title compound (20 mg); m.p. 159-162°C (from $CH_2Cl_2$-$Et_2O$), $\lambda$ max (MeCN) 335 ( 20,766) and 264 nm (12,395); $\nu$ max. (KBr) 1780, 1697 and 1678 $cm^{-1}$; $\delta$ (DMF-$d_7$) 1.33 (3H, d, J 6.5 Hz, MeCH), 2.30 (3H, s, MeCO), 3.62 (1H, dd, J 10 and 18 Hz, 4-CHa), 3.74 (1H, dd, J 6 and 9.5 Hz, 6-CH), 3.76 (H, dd, J 8.5 and 18 Hz, 4-$CH_6$), 4.18 (1H, m, CHMe), 4.46 (1H, m, 5-CH), 5.22 (1H, br, OH), 5.43 and 5.60 (each 1H, d, J 14 Hz, $CH_2Ar$), 6.55 (1H, d, J 16 Hz, =CH.CO), 7.85 and 8.32 (each 2H, d, J 8.5 Hz, $C_6H_4$-$NO_2$), and 8.03 (1H, d, J 16 Hz, =CHS). [Found: C, 55.5; H, 4.6; N, 6.45%; $M^+$, 432.1022 : $C_{20}H_{20}N_2O_7S$ requires C, 55.55; H, 4.65; N, 6.5%, M, 432.0991].

Further elution gave the title (Z)-isomer (56 mg); m.p. 138-141° (from $CH_2Cl_2$-$Et_2O$); $\lambda$ max (MeCN) 340 ( 19,142) and 264 nm (11,767); $\nu$ max. (KBr) 1780, 1705,

1680 and 1665 $cm^{-1}$; δ (DMF-$d_7$) 1.33 (3H, d, J 6.5 Hz, MeCH), 2.27 (3H, s, MeCO), 3.56 (1H, dd, J 10 and 18 Hz, 4-CHa), 3.72 (1H, dd, J 6 and 9.5 Hz, 6-CH), 3.73 (1H, dd, J 9 and 18 Hz, 4-$CH_b$), 4.18 (1H, m, CHMe), 4.43 (1H, m, 5-CH), 5.18 (1H, br, OH), 5.43 and 5.60 (each 1H, d, J 14 Hz, $CH_2Ar$), 6.66 (1H, d, J 10 Hz, =CHCO), 7.70 (1H, d, J 10 Hz, =CHS), 7.89 and 8.32 (each 2H, d, J 9 Hz, $CH_2C_6H_4$-$NO_2$); [Found: C, 55.3; H, 4.7; N, 6.15%; $M^+$, 432.1013: $C_{20}H_{20}N_2O_7S$ requires C, 55.55; H, 4.65; N, 6.5%; M, 432.0991].

Preparation 8

p-Nitrobenzyl(5R,6R)-3-[(Z)-3-oxobut-1-en-1-ylthio]-6-[(S)-1-trimethylsilyloxyethyl]-7-oxo-1-azabicyclo-[3.2.0]hept-2-ene-2-carboxylate

p-Nitrobenzyl(5R,6R)-3-[(Z)-3-oxo-but-1-en-1-ylthio]-6-[(S)-1-hydroxyethyl]-7-oxo-1-azabicyclo-[3.2.0]hept-2-ene-2-carboxylate (300 mg) was treated with trimethylsilyl chloride (0.26 ml) in pyridine (3 ml) as described in Preparation 1. The title product was obtained as a solid (228 mg); m.p. 163-165°C (from ethyl acetate - $Et_2O$); λmax. (MeCN) 343 (22,050), and 265 nm (13,625); νmax ($CH_2Cl_2$) 1785, 1710 $cm^{-1}$; δ ($CDCl_3$) 0.09 (9H, s, $Me_3Si$), 1.34 (3H, d, J 6.5 Hz, MeCH), 2.29 (3H, s, MeCO), 3.17 (1H, dd, J 10.5 and 17.5 Hz, 4-CHa), 3.63 (1H, overlapping dd, J 6 and 6 Hz, 6-CH), 3.91 (1H, dd, J 8.5 and 17.5 Hz, 4-$CH_b$), 4.34 (2H, m, 5-CH and CHMe), 5.33 and 5.52 (each 1H, d, J 13.5 Hz, $CH_2Ar$), 6.46 (1H, d, J 10 Hz, =CHCO), 7.22 (1H, d, J 10 Hz, =CHS), 7.2 and 8.25 (each 2H, d, J 9 Hz, $C_6H_4$-$NO_2$); [Found: C, 54.5; H, 5.7; N, 5.6%; $M^+$, 504.1359. $C_{23}H_{28}N_2O_7SSi$ requires C, 54.75; H, 5.6; N, 5.55%; M, 504.1386].

Preparation 9

p-Nitrobenzyl (5R,6R)-3-(3-hydroxybut-1-en-1-ylthio)-6-[(S)-1-trimethylsilyloxyethyl]-7-oxo-1-azabicyclo-[3.2.0]hept-2-ene-2-carboxylate

A solution of p-Nitrobenzyl (5R,6R)-3-[(Z)-3-oxo-but-1-en-1-ylthio]-6-[(S)-1-trimethylsilyloxyethyl]-7-oxo-1-azabicyclo[3.2.0]hept-2-ene-2-carboxylate (170 mg) in THF (10 ml) was cooled to -20° and a solution of sodium borohydride (40 mg) in water (1 ml) was added. The solution was stirred at -20° and when all the starting material had been consumed (by t.l.c.) (ca. 45 min) ethyl acetate was added. The solution was washed with water and brine, then dried ($MgSO_4$) and evaporated in vacuo. Chromatography of the crude product on silica gel using a gradient elution of 30-50% hexane in ethyl acetate afforded the title compound as a clear oil (83 mg); $\lambda$ max (EtOH) 325 and 265 nm; max. ($CH_2Cl_2$) 3580, 1780 and 1700 $cm_{-1}$.

Preparation 10

p-Nitrobenzyl(5R,6R)-3-(3-azidobut-1-en-1-ylthio)-6-[(S)-1-trimethylsilyloxyethyl]-7-oxo-1-azabicyclo-[3.2.0]hept-2-ene-2-carboxylate

p-Nitrobenzyl (5R,6R)-3-(3-hydroxybut-1-en-1-ylthio)-6-[(S)-1-trimethylsilyloxyethyl]-7-oxo-1-azabicyclo[3.2.0]hept-2-ene-2-carboxylate (105 mg) was allowed to react with triphenylphosphine, di-isopropylazodicarboxylate and hydrazoic acid in THF as described in Preparation 3. After 30 minutes the reaction was complete, and work-up and chromatography again in the manner of Preparation 3 afforded the title azido derivative (41 mg); $\lambda$max. (EtOH) 324 and 264 nm; $\nu$ max. ($CH_2Cl_2$) 2110, 1785 and 1705 $cm^{-1}$.

Preparation 11

p-Nitrobenzyl (5R,6S)-3-[(Z)-3-oxo-but-1-en-1-ylthio]-6-[(R)-1-formyloxyethyl]-7-oxo-1-azabicyclo[3.2.0]hept-2-ene-2-carboxylate

Using the methodology of preparation 7, p-Nitrobenzyl (5R,6S)-3-[(E)-2-acetamidoethenylthio]-6-[(R)-1-formyloxyethyl]-7-oxo-1-azabicyclo[3.2.0]hept-2-ene-2-carboxylate (300 mg) was treated successively with N-bromoacetamide and but-3-yn-2-one to furnish the title ketone (80 mg); m.p. 151-154$^{o}$ (from ethyl acetate-hexane); $\lambda$ max. (MeCN) 342 ( 18964) and 265 nm (11,467); $\nu$ max. (KBr) 1780, 1720-1710 and 1665 $cm^{-1}$; $\delta$(DMF-$d_7$) 1.40 (3H, d, J 6 Hz, MeCH), 2.27 (3H, s, MeCH), 2.27 (3H, s, MeCO), ca. 3.55 (1H, m, 4-CHa), 3.73 (1H, dd, J 10 and 18 Hz, 4-$CH_b$), 3.94 (1H, dd, J 3 and 6 Hz, 6-CH), 4.42 (1H, m, 5-CH), 5.39 (1H, m, CHMe), 5.45 and 5.62 (each 1H, d, J 14 Hz, $CH_2Ar$), 6.66 (1H, d, J 10 Hz, =CHCO), 7.65 (1H, d, J 10 Hz, =CHS), 7.89 and 8.32 (each 2H, d, J 9 Hz, $C_6H_4$-$NO_2$), 8.35 (1H, s, OCHO); [Found: C, 54.6; H, 4.3; N, 6.0%. $C_{21}H_{20}H_2O_8S$ requires C, 54.8; H, 4.35; N, 6.1%].

Preparation 12

p-Nitrobenzyl (5R,6S)-3-(3-hydroxybut-1-en-1-ylthio)-6-[(R)-1-formyloxyethyl]-7-oxo-1-azabicyclo[3.2.0]-hept-2-ene-2-carboxylate

The reduction of p-Nitrobenzyl (5R,6S)-3-[(Z)-3-oxobut-1-en-1-ylthio]-6-[(R)-1-formuloxyethyl]-7-oxo-1-azabicyclo[3.2.0]hept-2-ene-2-carboxylate (500 mg) with sodium borohydride was accomplished by the method described in Preparation 9. The title hydroxy-compound was obtained as a yellow foam (131 mg); $\lambda$ max. (EtOH) 324 and 266 nm; $\nu$ max. ($CH_2Cl_2$) 3600, 1790, 1725 and 1710sh $cm^{-1}$; $\delta$ ($CDCl_3$) 1.29 (3H, d, J 7 Hz, MeCH.C=),

1.45 (3H, d, *J* 6.5 Hz, MeCHO), 2.03 (1H, br, OH), *ca*. 3.12 (1H, dd, *J* 8.5 and 18 Hz, 4-CHa), 3.34 (1H, dd, *J* 10 and 18 Hz, 4-$CH_b$), 3.40 (1H, dd, *J* 3 and 7 Hz, 6-CH), 4.23 (1H, m, 5-CH), 4.82 (1H, m, MeCHC=), 5.26 and 5.52 (each 1H, d, *J* 13.5 Hz, $CH_2Ar$), 5.41 (1H, m, MeCHO), 5.96 (1H, dd, *J* 9 and 8 Hz, =CH.CH), 6.2 (1H, d, *J* 9 Hz, SCH=), 7.66 and 8.23 (each 2H, d, *J* 9 Hz, $C_6H_4$-$NO_2$) and 8.06 (1H, d, OCHO).

## Preparation 13

## p-Nitrobenzyl(5R,6S)-3-(3-azidobut-1-en-1-ylthio)-6-[(R)-1-formyloxyethyl]-7-oxo-1-azabicyclo[3.2.0]-hept-2-ene-2-carboxylate

The reaction of *p*-Nitrobenzyl (5*R*,6*S*)-3-(3-hydroxybut-1-en-1-ylthio)-6-[(*R*)-1-formyloxyethyl]-7-oxo-1-azabicyclo[3.2.0]hept-2-ene-2-carboxylate (180 mg) with hydrazoic acid, triphenylphosphine and di-isopropylazodicarboxylate was performed as in Preparation 3. Work-up after 15 minutes followed by chromatography on silica-gel using 30-60% ethylacetate in hexane to elute gave a partially purified product which was rechromatographed using chloroform to elute to afford the title azide as an oil (30 mg); $\lambda$ max. (EtOH) 321 and 264 nm.; $\nu$ max. ($CH_2Cl_2$) 1790, 1730 and 1705sh $cm^{-2}$.

## Example 3

## (5R,6R)-3-(3-Aminobut-1-en-1-ylthio)-6-[(S)-1-hydroxyethyl]-7-oxo-1-azabicyclo[3.2.0]hept-2-ene-2-carboxylic acid

Following the procedure in Example 1, *p*-Nitrobenzyl (5*R*,6*R*)-3-(3-azidobut-1-en-1-ylthio)-6-[(*S*)-1-trimethylsilyloxyethyl]-7-oxo-1-azabicyclo[3.2.0]-hept-2-ene-2-carboxylate (70 mg) was converted into the title amino acid; $\lambda$ max. ($H_2O$) 301 nm.

Example 4

(5R,6S)-3-(3-Aminobut-1-en-1-ylthio)-6-[(R)-1-formyloxyethyl]-7-oxo-1-azabicyclo[3.2.0]hept-2-ene-2-carboxylic acid

p-Nitrobenzyl (5R,6S)-3-(3-azidobut-1-en-1-ylthio)-6-[(R)-1-formyloxyethyl]-7-oxo-1-azabicyclo-[3.2.0]hept-2-ene-2-carboxylate (20 mg) was shaken with hydrogen and 5% Pd-C in a mixture of dioxan (10 ml) and 0.05 M pH 7 phosphate buffer solution (5 ml). After 2.5 h the mixture was filtered over Celite, washing with water, and the aqueous solution then washed (x3) with ethyl acetate. Concentration of the aqueous solution followed by chromatography on Diaion HP20 eluting with water and 10% ethanol-water afforded an aqueous solution of the title amino acid; $\lambda$ max. ($H_2O$) 301 nm.

Example 5

(5R,6R)-3-[(Z)-3-Acetimidoylaminoprop-1-enylthio]-6-[(S)-1-hydroxyethyl]-7-oxo-1-azabicyclo[3.2.0]hept-2-ene-2-carboxylic acid

An aqueous solution of (5R,6R)-3-[(Z)-3-aminoprop-1-enylthio]-6-[(S)-1-hydroxyethyl]-7-oxo-1-azabicyclo-[3.2.0]hept-2-ene-2-carboxylic acid (12 mg in 5 ml) was cooled in an ice bath with stirring. The pH of the solution was adjusted to 9.0 by the addition of dilute sodium hydroxide solution. Ethyl acetimidate hydrochloride (101 mg) was added portionwise over a period of 5 minutes, whilst maintaining the pH of the solution at 9.0. Stirring was continued for 30 minutes. The pH of the solution was then readjusted to 7.0 by the addition of dilute hydrochloric acid and chromatographed over HP-20, eluting with a gradient of 0-10%

ethanol/water. The fractions were monitored by u.v. spectroscopy and h.p.l.c. Those fractions containing the title compound were combined and lyophilised to yield a white solid, λ max. ($H_2O$) 302 nm, νmax. (KBr) 3300 (broad), 1750, 1685, 1640, 1585 $cm^{-1}$.

Example 6

(5R,6R)-3-[(Z)-3-Formimidoylaminoprop-1-enylthio]-6-[(S)-1-hydroxyethyl]-7-oxo-1-azabicyclo[3.2.0]hept-2-ene-2-carboxylic acid

The title compound was prepared as a white solid by reaction of (5R,6R)-3-[(Z)-3-aminoprop-1-enylthio]-6-[(S)-1-hydroxyethyl]-7-oxo-1-azabicyclo[3.2.0]hept-2-ene-2-carboxylic acid (see Example 1) with ethyl formimidate hydrochloride, by the procedure described in Example 5, λ max ($H_2O$) 303 nm.

Example 7

(5R,6S)-3-(3-Aminobut-1-en-1-ylthio)-6-[(R)-1-hydroxyethyl]-7-oxo-1-azabicyclo[3.2.0]hept-2-ene-2-carboxylic acid

An aqueous solution of (5R,6S)-3-(3-aminobut-1-en-1-ylthio)-6[(R)-1-formyloxyethyl]-7-oxo-1-azabicyclo[3.2.0]hept-2-ene-2-carboxylic acid was prepared as in Example 4. The pH of the solution was adjusted to 8.5 with 0.1N NaOH, and the solution maintained at this pH at 37°C for 2 h. The solution was neutralised to pH7 with 0.1N HCl and concentrated to small volume (10 ml), before loading onto a column of Diaion HP-20. Elution with water, followed by 5% ethanol-water afforded an aqueous solution of the title amino acid; λ max ($H_2O$) 304 nm.

Preparation 14

p-Nitrobenzyl (5R,6R)-3-[(Z)-3-oxo-1-methylprop-1-enylthio]-6-[(S)-1-hydroxyethyl]-7-oxo-1-azabicyclo[3.2.0]-hept-2-ene-2-carboxylate

A. 2-Butyn-1-ol (1.0 gm) was dissolved in dry N,N-dimethylformamide (20 ml) and stirred vigourously for 16 hours at room temperature with 'Attenburrow' manganese dioxide (5 gm). After removal of the manganese dioxide by centrifuging, the crude solution of 2-butyn-1-al was used in the next step.

B. p-Nitrobenzyl (5R,6R)-3-[(E)-2-acetamidovinyl thio]-6-[(S)-1-hydroxyethyl]-7-oxo-1-azabicyclo[3.2.0]-hept-2-ene-2-carboxylate (0.250 gm) was dissolved in 1,4-dioxan (10 ml) containing water (15 drops). A solution of N-bromoacetamide (0.078 gm) in 1,4-dioxan (5 ml) was added and the solution was stirred at room temperature for 5 minutes. Chloroform (100 ml) was added and the organic solution washed with pH 7.0 phosphate buffer, brine and dried over $MgSO_4$. Removal of the solvent at reduced pressure afforded the crude C-3 thiol as a colourless oil. This oil was dissolved in the solution of but-2-yn-1-al in N,N-dimethylformamide obtained from part A. Anhydrous potassium carbonate (0.039 gm) was added and the solution stirred at room temperature for 20 min. The solution was then partitioned between ethyl acetate and brine. The organic layer was dried over $MgSO_4$ and the solvent removed at reduced pressure to afford the crude product, which was chromatographed over silica gel (20 gm). Elution with 80% ethyl acetate/hexane gave a mixture of p-nitrobenzyl (5R,6R)-3-[(Z)-3-oxo-1-methylprop-1-enylthio]-6-[(S)-1-hydroxyethyl]-7-oxo-1-azabicyclo[3.2.0]hept-2-ene-2-carboxylate and the corresponding (E)-isomer in the approximate ratio of 5:1, as a pale yellow gum (0.159 gm), $\nu$ max ($CHCl_3$)

3450, 1785, 1720, 1681, 1612, 1563 and 1523cm$^{-1}$; λmax (EtOH) 317, 264 nm; $\delta_H$ ($CDCl_3$) 1.40 (3H, d, J 6.5Hz, $CH_3CH$), 2.32 (major) and 2.46 (minor) (3H, d, J 1 Hz, $CH_3C$=C), 2.9-3.2 (1H, 2 x dd, major and minor isomers, 4-Ha), 3.45-3.75 (2H, dd + m, 4-$H_b$ + 6-H), 4.15-4.50 (2H, m, 5-H + 8-H), 5.28+5.51 (2H, 2 x d, $CH_2Ar$), 6.36 (major isomer) + 6.00 (minor isomer) (1H, dd, J 1Hz and 7Hz, C=CH), 7.64 (2H, d, aromatic protons), 8.24 (2H, d, aromatic protons), 10.08 (major isomer) and 9.83 (minor isomer) (1H, d, J 7Hz, CHO).

## Preparation 15

### p-Nitrobenzyl (5R,6R)-3-[(Z)-3-oxo-1-methylprop-1-enylthio]-6-[(S)-1-trimethylsilyloxyethyl]-7-oxo-1-azabicyclo[3.2.0]hept-2-ene-2-carboxylate

The mixture of p-nitrobenzyl (5R,6R)-3-[(Z)-3-oxo-1-methylprop-1-enylthio]-6-[(S)-1-hydroxyethyl]-7-oxo-1-azabicyclo[3.2.0]hept-2-ene-2-carboxylate and the corresponding (E)-isomer (0.130 gm) was dissolved in pyridine (2 ml) and stirred at room temperature for one hour with trimethylsilyl chloride (0.196 gm). The solvent was then evaporated at reduced pressure and the residue partitioned between ethyl acetate and water. The organic layer was washed with brine, dried over anhydrous magnesium sulphate and evaporated at reduced pressure to yield the crude product, which was chromatographed over silica gel. Elution with 1:1 ethyl acetate/hexane afforded p-nitrobenzyl (5R,6R)-3-[(Z)-3-oxo-1-methylprop-1-enylthio]-6-[(S)-1-trimethylsilyloxyethyl]-7-oxo-1-azabicyclo[3.2.0] hept-2-ene-2-carboxylate, slightly contaminated with the (E)-isomer (0.093 gm), νmax ($CHCl_3$) 1788, 1722, 1678, 1610, 1562, 1522, 842cm$^{-1}$; λmax (EtOH) 318, 263 nm, $\delta_H$ ($CDCl_3$) 0.11 (9H, s, $Me_3$), 1.31 (3H, s, J 6.5Hz, $CH_3CH$), 2.29 (major isomer) and 2.46 (minor

isomer) (3H, d, J ca. 1Hz, $CH_3C=C$), 2.65-3.25 (1H, m, 4-Ha), 3.45-3.95 (2H, m, 4-$H_b$ + 6-H), 4.1-4.5 (2H, m, 5-H + 8-H), 5.26 + 5.52 (2H, 2 x d, $CH_2Ar$), 6.34 (major isomer) + 5.96 (minor isomer) (1H, broad d, C=CH), 7.64 (2H, d, aromatic H), 8.20 (2H, d, aromatic H), 10.15 (major isomer) and 9.91 (minor isomer) (1H, d, J 7Hz, CHO).

Preparation 16

p-Nitrobenzyl (5R,6R)-3-[(Z)-3-hydroxy-1-methyl-prop-1-enylthio]-6-[(S)-1-trimethylsilyloxyethyl]-7-oxo-1-aza-bicyclo[3.2.0]hept-2-ene-2-carboxylate

The mixture of p-nitrobenzyl (5R,6R)-3-[(Z)-3-oxo-1-methylprop-1-enylthio]-6-[(S)-1-trimethylsilyloxy-ethyl]-7-oxo-1-azabicyclo[3.2.0]hept-2-ene-2-carboxylate and its (E)-isomer (0.090 gm) was dissolved in tetrahydrofuran (10 ml) and cooled to -40°C. A solution of sodium borohydride (0.021 gm) in 20% aqueous tetrahydrofuran (10 ml) was added and stirring was continued at this temperature for 15 minutes. The solution was then partitioned between ethyl acetate and water. The organic layer was washed with brine, dried over anhydrous magnesium sulphate and evaporated at reduced pressure. The residual gum was chromatographed over silica gel (10 gm). Elution with a gradient of 25-50% EtOAc/hexane afforded the pure p-nitrobenzyl (5R,6R)-3-[(Z)-3-hydroxy-1-methylprop-1-enylthio]-6-[(S)-1-trimethylsilyloxyethyl]-7-oxo-1-azabicyclo[3.2.0]-hept-2-ene-2-carboxylate (0.045 gm) as a colourless oil, max ($CHCl_3$) 1786, 1709, 1612, 1558, 1522 and 848$cm^{-1}$, $\lambda$ max (EtOH) 316, 267 nm, $\delta_H$ ($CDCl_3$) 0.11 (9H, s, $Me_3Si$), 1.32 (3H, d, J 6.5 Hz, $CH_3CH$), 1.81 (1H, broad res., OH), 2.13 (3H, d, J 1Hz, $CH_3C=C$), 2.87 (1H, dd, J 10Hz and 18Hz, 4-Ha), 3.45-3.65 (2H, dd + t, J 9Hz, 18Hz and 6Hz, 4-$H_b$ + 6-H), 4.20-4.32 (2H, m, 5-H

+ 8-H), 4.36 (2H, dd, J 7Hz and ca. 1Hz, $C=CCH_2$), 5.27 and 5.51 (2H, 2xd, J 14.5Hz, $CH_2Ar$) 6.21 (1H, dt, J 1.5 and 6.5Hz, C=CH), 7.67 and 8.22 (4H, 2xd, aromatic protons),

Preparation 17

p-Nitrobenzyl(5R,6R)-3-[(Z)-3-azido-1-methylprop-1-enylthio]-6-[(S)-1-trimethylsilyloxyethyl]-7-oxo-1-azabicyclo[3.2.0]hept-2-ene-2-carboxylate

p-Nitrobenzyl (5R,6R)-3-[(Z)-3-hydroxy-1-methylprop-1-enylthio]-6-[(S)-1-trimethylsilyloxyethyl]-7-oxo-1-azabicyclo[3.2.0]hept-2-ene-2-carboxylate (0.045 gm) was dissolved in dry tetrahydrofuran (20 ml) and cooled to 0°C with triphenylphosphine (0.047 gm). Hydrazoic acid (1.7M solution in toluene, 0.21 ml) was added followed by diisopropylazodicarboxylate (0.036 gm). Stirring was continued for 15 minutes at room temperature. Ethyl acetate was then added and the organic solution was washed with dilute sodium bicarbonate solution, brine and dried over anhydrous magnesium sulphate. Removal of the solvent at reduced pressure afforded an oil, which was chromatographed over silica gel (8 gm). Elution with 25% ethyl acetate/hexane afforded the title compound as a colurless oil (0.022 gm), $\nu_{max}$ ($CHCl_3$) 2100, 1780, 1703, 1608, 1558, 1521 and 841 $cm^{-1}$, $\lambda_{max}$ (EtOH) 315, 266 nm, $\delta_H$ ($CDCl_3$) 0.11 (9H, s, $Me_3Si$), 1.32 (3H, d, J 6.5Hz, $CH_3CH$), 2.18 (3H, d, J 1Hz, $CH_3C=C$), 2.84 (1H, dd, J 10Hz and 18Hz, 4-Ha), 3.58 (1H, dd, J 9Hz and 18Hz, 4-$H_b$), 3.60 (1H, t, J 7Hz, 6-H), 4.09 (2H, m, $CH_2N_3$), 4.2-4.35 (2H, m, 5-H + 8-H), 5.27 and 5.52 (2H, 2 x d, J 14Hz, $CH_2Ar$), 6.10 (1H, dt, J 1Hz and 7 1Hz, C=CH), 7.67 and 8.21 (4H, 2 x d, J 9Hz, aromatic protons).

Example 8

(5R,6R)-3-[(Z)-3-amino-1-methylprop-1-enylthio]-6-[(S)-1-hydroxyethyl]-7-oxo-1-azabicyclo[3.2.0]hept-2-ene-2-carboxylic acid

The title compound was prepared as an aqueous solution by hydrogenolysis of p-nitrobenzyl (5R,6R)-3-[(Z)-3-azido-1-methylprop-1-enylthio]-6-[(S)-1-trimethylsilyloxyethyl]-7-oxo-1-azabicyclo[3.2.0]hept-2-ene-2-carboxylate, following the procedure outlined in example 1, $\lambda$ max ($H_2O$) 297 nm.

Preparation 18

p-Nitrobenzyl(5R,6R)-3-[3-oxo-1-phenylprop-1-enylthio]-6-[(S)-1-hydroxyethyl]-7-oxo-1-azabicyclo[3.2.0]hept-2-ene-2-carboxylate

Using the methodology of Preparation 7, p-Nitrobenzyl (5R,6R)-3-[(E)-2-acetamidoethenylthio]-6-[(S)-1-hydroxyethyl]-7-oxo-1-azabicyclo[3.2.o]hept-2-ene-2-carboxylate (0.3 g) was treated successively with N-bromoacetamide (0.093 g) and 3-phenylprop-2-ynal (0.131 g) to afford the title aldehyde (0.155 g) as a mixture (ca. 2:5:1) of (Z)- and (E)- isomers; $\nu$ max ($CH_2Cl_2$) 3600, 1790, 1725 and 1670 $cm^{-1}$; $\delta$ ($CDCl_3$) (Z)-isomer, 1.28 (3H, d, J 6.5 Hz, MeCH), 1.47 (1H, d, OH), 2.48 (1H, dd, J 19 and 10.5 Hz, 4-CHa), 3.03 (1H, dd, J 19 and 9Hz, 4-$CH_b$), 3.45 (1H, dd, J 6 and 8.5 Hz, 6-CH), 3.85 (1H, m, CHMe), 4.00 (1H, m, 5-CH), 5.31 and 5.56 (each 1H, d, J 13.5Hz, $CH_2Ar$), 6.65 (1H, d, J 7.5Hz, =CH), 7.48 (5H, m, Ph), 7.66 and 8.28 (each 2H, m, $ArCH_2$) and 10.30 (1H, d, J 7.5Hz, CHO); (E)-isomer, 1.33 (3H, d, J 6.5 Hz, MeCH), 1.72 (1H, d, OH), 2.77 (1H, dd, J 19 and 10.5Hz, 4-CHa), 3.23 (1H, dd, J 9 and 19Hz, 4-$CH_b$), 3.54 (1H, dd, J 6 and 8.5Hz, 6-CH), 4.00

(1H, m, CHMe), 4.14 (1H, m, 5-CH), 5.28 and 5.53 (each 1H, d, *J* 13.5Hz, $CH_2Ar$), 6.42 (1H, d, *J* 7.5Hz, =CH), 7.65 (5H, m, Ph), 7.65 and 8.25 (each 2H, m, $ArCH_2$) and 9.38 (1H, d, *J* 7.5Hz, CHO).

Preparation 19

p-Nitrobenzyl(5R,6R)-3-(3-hydroxy-1-phenylprop-1-en-1-ylthio)-6-[(S)-1-trimethylsilyloxyethyl]-7-oxo-1-azabicyclo[3.2.0]hept-1-ene-2-carboxylate

*p*-Nitrobenzyl (5R,6R)-3-[3-oxo-1-phenylprop-1-enylthio]-6-[(S)-1-hydroxyethyl]-7-oxo-1-azabicyclo 3.2.0]hept-2-ene-2-carboxylate (0.133 g) was treated with trimethylsilyl chloride (0.1 ml) in pyridine (5 ml) in the manner described in Preparation 1. After work-up the crude product was dissolved in tetrahydrofuran, and the solution treated with sodium borohydride (28 mg) as in Preparation 16. After work-up, the crude product was chromatographed using 30% hexane in ethyl acetate as eluant to afford the title alcohol (101 mg); [(Z)-isomer:(E)-isomer=*ca*. 2:1]; $\lambda$ max (EtOH) 321 and 254 nm; $\nu$ max ($CH_2Cl_2$) 3610, 1790 and 1705$cm^{-1}$; $\delta$ ($CDCl_3$), (Z)-isomer, 0.03 (9H, s, $Me_3Si$), 1.24 (3H, d, *J* 6.5Hz, MeCH), 1.64 (1H, br, OH), 2.57 (1H, dd, *J*18 and 10Hz, 4-CHa), 3.12 (1H, dd, *J* 18 and 9Hz, 4-$CH_b$), 3.48 (1H, dd, *J* 6 and 7.5Hz, 6-CH), 4.02 (2H, m, 5-CH and CHMe), 4.62 (2H, m, $H_2$CH=), 5.28 and 5.56 (each 1H, d, *J* 13.5Hz, $ArCH_2$), 6.62 (1H, t, *J* 6Hz, CH=), 7.37 (5H, m, Ph), 7.67 and 8.23 (each 2H, d, *J* 9Hz, $ArCH_2$); (*E*)-isomer similar except 0.06 (9H, s, $Me_3Si$), 2.62 (1H, 4-CHa), 3.17 (1H, 4-Chb), 3.46 (6-CH), 4.27 (2H, d, *J* 6.5Hz, =CH.$CH_2$), 7.58 (5H, Ph), 7.65 and 8.22 (each 2H, $ArCH_2$).

Preparation 20

p-Nitrobenzyl (5R,6R)-3-(3-azido-1-phenylprop-1-enylthio)-6-[(S)-1-trimethylsilyloxyethyl]-7-oxo-1-azabicyclo[3.2.0]hept-2-ene-2-carboxylate

p-Nitrobenzyl (5R,6R)-3-(3-hydroxy-1-phenylprop-1-enylthio)-6-[(S)-1-trimethylsilyloxyethyl]-7-oxo-1-azabicyclo[3.2.0]hept-2-ene-2-carboxylate (85 mg) was treated with triphenylphosphine (90 mg), di-isopropylazodicarboxylate (69 mg) and hydrazoic acid (0.1 ml of a 1.7M solution in toluene) in the manner described in Preparation 17. Chromatography of the crude product using 25–35% ethylacetate in hexane to elute gave the title azido-derivative as a clear oil (41 mg) [(Z):(E)-isomers=ca. 2.5:1]; $\lambda$max (EtOH) 321 and 256nm; max ($CH_2Cl_2$) 2100, 1785 and 1710 $cm^{-1}$; $\delta$ ($CDCl_3$); (Z)-isomer, 0.03 (9H, s, $Me_3Si$), 1.22 (3H, d, J 6.5Hz, MeCH), 2.52 (1H, dd, J 10 and 18 Hz, 4-CHa), 3.12 (1H, dd, J 9 and 18 Hz, 4-$CH_b$), 3.47 (1H, dd, J 6 and 7.5Hz, 6-CH), 4.02 (2H, m, 5-CH and CHMe), 4.24 (1H, dd, J 6.5 and 14.5Hz, =$CHCH_a$), 4.37 (1H, dd, J 7.5 and 14.5 Hz, =CH.$CH_b$), 5.29 and 5.57 (each 1H, d, J 14Hz, $CH_2Ar$), 6.46 (1H, t, J 7Hz, $CHCH_2$), 7.40 (5H, m, Ph), 7.68 and 8.24 (each 2H, d, J 9Hz, $ArCH_2$); (E)-isomer similar except 0.06 (9H, s, $Me_3Si$), 1.24 (3H, d, MeCH), 2.62 (1H, 4-CHa), 3.23 (1H, 4-CHb), 3.48 (1H, 6-CH), ca. 4.0 (2H, m, =$CHCH_2$), 5.26 and 5.52, 2H, $CH_2Ar$), 6.44 (1H, =CH), 7.58 (5H, Ph), 7.67 and 8.22 (each 2H, $ArCH_2$).

Example 9

(5R,6R)-3-(3-Amino-1-phenylprop-1-enylthio)-6-[(S)-1-hydroxyethyl]-7-oxo-1-azabicyclo[3.2.0]hept-2-ene-2-carboxylic acid

The title amino-acid [as mixture (ca. 2.5:1) of (Z)- and (E)-isomers] was prepared in aqueous solution by hydrogenolysis of p-nitrobenzyl (5R,6R)-3-(3-azido-1-phenylprop-1-enylthio)-6-[(S)-1-trimethylsilyloxy-ethyl]-7-oxo-1-azabicyclo[3.2.0]hept-2-ene-2-carboxylate following the procedure outlined in example 1, except that the hydrogenation period was extended to 2.75h. The product possessed an absorption at $\lambda$ max ($H_2O$) 301 nm in the u.v. spectrum.

Preparation 21

p-Nitrobenzyl (5R,6S)-3-[(Z)-3-oxo-1-methylprop-1-enylthio]-6-[(R)-1-hydroxyethyl]-7-oxo-1-azabicyclo[3.2.0]hept-2-ene-2-carboxylate

The title compound was prepared from p-nitrobenzyl (5R,6S)-3-[(E)-2-acetamidovinylthio]-6-[(R)-1-hydroxyethyl]-7-oxo-1-azabicyclo[3.2.0]hept-2-ene-2-carboxylate by the procedure described in preparation 14, $\nu_{max}$ ($CHCl_3$) 3400, 1782, 1720, 1678, 1608 $cm^{-1}$; $\lambda_{max}$ (EtOH) 319, 263 nm; $\delta_H$ ($CDCl_3$) 1.36 (3H, d, *J* 6.3 Hz, $C\underline{H}_3CH$), 2.30 (3H, s, vinylic $CH_3$), 3.02 (1H, dd, *J* 9 and 18 Hz, 4-$H_a$) 3.17 (1H, dd, *J* 9.75 and 18 Hz, 4-$H_b$), 3.25 (1H, dd, *J* 6.5 and 2.9 Hz, 6-H), 4.2 - 4.4 (2H, m, 5-H and 8-H), 5.27 (1H, d) and 5.52 (1H, d) ($CH_2Ar$), 6.37 (1H, dd, *J* 1.25 Hz and 7 Hz, C$\underline{H}$=C), 7.67 (2H, d, aromatic), 8.23 (2H, d, aromatic), 10.10 (1H, d, *J* 7 Hz, C$\underline{H}$O); m/e 432.0995 ($M^+$, $C_{20}H_{20}N_2O_7S$ requires 432.0991).

Preparation 22

p-Nitrobenzyl (5R, 6S)-3-[(Z)-3-hydroxy-1-methylprop-1-enylthio]-6-[(R)-1-trimethylsilyloxy-ethyl]-7-oxo-1-azabicyclo[3.2.0]hept-2-ene-2-carboxylate

The title compound was prepared from p-nitrobenzyl (5R, 6S)-3-[(Z)-3-oxo-1-methylprop-1-enylthio]-6-[(R)-1-hydroxyethyl]-7-oxo-1-azabicyclo[3.2.0]hept-2-ene-2-carboxylate via p-nitrobenzyl (5R, 6S)-3-[(Z)-3-oxo-1-methylprop-1-enylthio]-6-[(R)-1-trimethylsilyloxyethyl]-7-oxo-1-azabicyclo[3.2.0]hept-2-ene-2-carboxylate by the procedures described in preparations 15 and 16; $\nu_{max}$ ($CHCl_3$) 1780, 1702 and 1610 $cm^{-1}$; $\lambda_{max}$ (EtOH) 318, 266 nm; $\delta_H$ ($CDCl_3$) 0.16 (9H, s, $Si\underline{Me}_3$), 1.28 (3H, d, J 6 Hz, $C\underline{H}_3CH$), 2.11 (3H, s, vinylic $CH_3$), 2.96 (1H, dd, J 9 and 18 Hz, $4-H_a$), 3.10 (1H, dd, J 9.5 and 18 Hz, $4-H_b$), 3.15 (1H, dd, J 2.7 and 6.3 Hz, 6-H), 4.1 - 4.5 (4H, m, 5-H + 8-H + $C\underline{H}_2OH$), 5.28 (1H, d,) and 5.51 (1H, d) ($C\underline{H}_2Ar$), 6.19 (1H, dt, J 1.5 and 6.5 Hz, vinylic C$\underline{H}$), 7.66 (2H, d, aromatic), 8.22 (2H, d, aromatic); m/e 506.1544 ($M^+$, $C_{23}H_{30}N_2O_7SSi$ requires 506.1543).

Preparation 23

p-Nitrobenzyl (5R, 6S)-3-[(Z)-3-azido-1-methylprop-1-enylthio]-6-[(R)-1-trimethylsilyl-oxyethyl]-7-oxo-1-azabicyclo[3.2.0]hept-2-ene-2-carboxylate

The title compound was prepared from *p*-nitrobenzyl (5R, 6S)-3-[(Z)-3-hydroxy-1-methylprop-1-enylthio]-6-[(R)-1-trimethylsilyloxyethyl]-7-oxo-1-azabicyclo[3.2.0] hept-2-ene-2-carboxylate by the procedure described in preparation 17, $\nu_{max}$ ($CHCl_3$) 2110, 1780, 1718, 1611 $cm^{-1}$; $\lambda_{max}$ (EtOH) 318, 266 nm; $\delta_H$ ($CDCl_3$) 0.14 (9H, s, $SiMe_3$), 1.30 (3H, d, $C\underline{H}_3CH$), 2.16 (3H, s, vinylic $C\underline{H}_3$), 2.93 (1H, dd, J 9 and 18 Hz, $4-H_a$), 3.03 (1H, dd, *J* 9.5 and 18 Hz, $4-H_b$), 3.15 (1H, dd, *J* 2.8 and 6.3 Hz, 6-H), 4.0 - 4.3 (4H, m, 5-H + 8-H + $C\underline{H}_2OH$), 5.25 (1H, d) and 5.49 (1H, d), ($C\underline{H}_2Ar$), 6.09 (1H, dt, *J* 1.35 and 7 Hz, vinylic C$\underline{H}$), 7.66 (2H, d, aromatic) 8.21 (2H, d, aromatic).

Example 10

(5R, 6S)-3-[(Z)-3-amino-1-methylprop-1-enylthio]-6-[(R)-1-hydroxyethyl]-7-oxo-1-azabicyclo[3.2.0]hept-2-ene-2-carboxylic acid

The title compound was prepared as an aqueous solution by hydrogenolysis of p-nitrobenzyl (5R, 6S)-3-[(Z)-3-azido-1-methylprop-1-enylthio]-6-[(R)-1-trimethylsilyloxyethyl]-7-oxo-1-azabicyclo[3.2.0]hept-2-ene-2-carboxylate, following the procedure outlined in example 1, $\lambda_{max}(H_2O)$ 300 nm.

Preparation 24

p-Nitrobenzyl (5R,6R)-3-[(E)-3-oxoprop-1-enylthio]-6-[(S)-1-hydroxyethyl]-7-oxo-1-azabicyclo[3.2.0]hept-2-ene-2-carboxylate

A solution of trimethylsilyl acetylene (1.4g) in dry tetrahydrofuran (30ml) at -25° under Argon was treated with n-butyllithium (9.2ml of a 1.55M solution in hexane). After 10 min. ethyl formate (2.3ml) was added to the reaction mixture, and after a further 15 min. the reaction was quenched with saturated ammonium chloride solution (50ml). The mixture was shaken vigorously and the upper layer retained (40ml). This contained a solution of 3-trimethylsilylprop-2-yn-1-al.

Using the methodology of Preparation 7, p-Nitrobenzyl (5R,6R)-3-[(E)-2-acetamidoethenylthio]-6-[(S)-1-hydroxyethyl]-7-oxo-1-azabicyclo [3.2.0]hept-2-ene-2-carboxylate (300mg) was treated with N-bromoacetamide and the product then allowed to react with the aforementioned solution of 3-trimethylsilylprop-2-yn-1-al (5ml) and anhydrous potassium carbonate (46mg) in DMF (10ml). Work-up as described followed by chromatography on silica gel using 20% and 10% hexane in ethyl acetate for elution afforded the title aldehyde (58mg); $\lambda_{max}$ (EtOH) 337 and 263 nm; $\nu_{max}$ ($CH_2Cl_2$) 1785, 1705 and 1675 $cm^{-1}$; $\delta$($CDCl_3$) 1.45 (3H, d, *J* 6.5 Hz, MeCH), 1.85 (1H, d, *J* 4.5 Hz, OH), 3.30 (1H, dd, J 18 and 10.5 Hz, 4-$CH_a$), 3.68 (1H, dd, *J* 6 and 8 Hz, 6-CH), 3.75 (1H, dd, *J* 18 and 8.5 Hz, 4-$CH_b$), 4.26 (1H, m, CHMe), 4.46 (1H, m, 5-CH), 5.28 and 5.51 (each 1H, d, *J*, 13.5 Hz, $CH_2$Ar), 6.44 (1H, dd, *J* 7 and 15.5 Hz, =CH.CHO), 7.73 (1H, d, *J* 15.5 Hz, =CHS), 8.24 and 7.65 (each 2H, d, *J* 13.5 Hz, Ar $CH_2$) and 9.48 (1H, d, *J* 7 Hz, CHO).

## Preparation 25

p-Nitrobenzyl (5R,6R)-3-[(E)-3-hydroxyprop-1-enylthio]-6-[(S)-1-trimethylsilyloxyethyl]-7-oxo-1-azabicyclo [3.2.0]hept-2-ene-2-carboxylate

p-Nitrobenzyl (5R,6R)-3-[(E)-3-oxoprop-1-enylthio]-6-[(S)-1-hydroxyethyl]-7-oxo-1-azabicyclo[3.2.0]hept-2-ene-2-carboxylate (55mg) was treated with trimethylsilyl chloride in the manner described in Preparation 1. The product (49mg) was then reduced with sodium borohydride (11mg) using the methodology of Preparation 16 to afford the title alcohol (28mg); $\lambda_{max}$ (EtOH) 324 and 264nm; $\nu_{max}$ ($CH_2Cl_2$) 3610, 1780 and 1700 $cm^{-1}$; $\delta$($CDCl_3$) 0.10 (9H, s, $Me_3Si$), 1.34 (3H, d, J 6.5 Hz, MeCH), 3.07 (1H, dd, J 10 and 18 Hz, 4-$CH_a$), 3.60 (1H, t, J 6 Hz, 6-CH), 3.69 (1H, dd, J 8.5 and 18 Hz, 4-$CH_b$), ca. 4.3 (4H, m, 5-CH, CHMe and $CH_2CH$=), 5.26 and 5.50 (each 1H, d, J 14 Hz, $CH_2Ar$), 6.21 (1H, dt, J 15 and 5 Hz, =CH.$CH_2$), 6.50 (1H, dt, J 15 and 1.5 Hz, SCH=), 7.66 and 8.22 (each 2H, d, J 9 Hz $ArCH_2$), $M^+$, 492.1388 ($C_{22}H_{28}N_2O_7SSi$ requires 492.1386).

Preparation 26

p-Nitrobenzyl (5R,6R)-3-[(E)-3-azidoprop-1-enylthio]-6-[(S)-1-trimethylsilyloxyethyl]-7-oxo-1-azabicyclo[3.2.0]hept-2-ene-2-carboxylate

p-Nitrobenzyl (5R,6R)-3-[(E)-3-hydroxyprop-1-enylthio]-6-[(S)-1-trimethylsilyloxyethyl]-7-oxo-1-azabicyclo[3.2.0]hept-2-ene-2-carboxylate (100mg) was treated with triphenylphosphine (90mg), di-isopropylazodicarboxylate (69mg) and hydrazoic acid (0.1ml of a 1.7M solution in toluene) using the methodology of Preparation 17. The title azido-derivative was obtained as an oil (62mg); $\lambda_{max}$ (EtOH) 321 and 263nm; $\nu_{max}$ ($CH_2Cl$) 2010, 1785 and 1705 $cm^{-1}$; $\delta(CDCl_3)$ 0.10 (9H, s, $Me_3Si$), 1.34 (3H, d, J 6 Hz, MeCH), 3.07 (1H, dd, J 10.5 and 18 Hz, 4-$CH_a$), 3.61 (1H, t, J 6 Hz, 6-CH), 3.73 (1H, dd, J 8.5 and 18 Hz, 4-$CH_b$), 3.90 (2H, d, J 5.5 Hz, =CH $CH_2$), 4.31 (2H, m, 5-CH and CHMe), 5.27 and 5.50 (each 1H, d, J 14, $CH_2Ar$), 6.09 (1H, dt, J 6 and 15 Hz, =CH $CH_2$), 6.54 (1H, d, J 15Hz, SCH=), 7.66 and 8.22 (each 2H, d, J 8.5 Hz, $C_6H_4.NO_2$).

Example 11

(5R,6R)-3-[(E)-3-aminoprop-1-enylthio]-6-[(S)-1-hydroxyethyl]-7-oxo-1-azabicyclo[3.2.0]hept-2-ene-2-carboxylic acid

The process described in Example 1 was employed to convert p-nitrobenzyl (5R,6R)-3-[(E)-3-azidoprop-1-enylthio]-6-[(S)-1-hydroxyethyl]-7-oxo-1-azabicyclo [3.2.0]hept-2-ene-2-carboxylate into the title amino acid; $\lambda_{max}$ ($H_2O$) 301nm.

Preparation 27

*p*-Nitrobenzyl (5R,6S)-3-[(*E*)-3-oxoprop-1-enylthio]-6-[(R)-1-formyloxyethyl]-7-oxo-1-azabicyclo[3.2.0]hept-2-ene-2-carboxylate

*p*-Nitrobenzyl (5R,6S)-3-[(*E*)-2-acetamidoethenylthio]-6-[(R)-1-formyloxyethyl]-7-oxo-1-azabicyclo[3.2.0]hept-2-ene-2-carboxylate (1g) was treated with N-bromoacetamide and the resulting product with 3-trimethylsilylprop-2-yn-1-al in an analogous way to Preparation 24. The title aldehyde was obtained as a solid (331mg) which was crystallised from hexane-ethyl acetate; m.p. 138-140$^{o}$C; $\lambda_{max}$ (MeCN) 337 (ε27,250) and 265 nm (14,367); $\nu_{max}$ (KBr) 1780, 1720, 1710sh and 1665 cm$^{-1}$; δ($CDCl_3$/DMF-$d_7$ 1.42 (3H, d, *J* 6.5 Hz, *Me*CH), 3.55 (1H, dd, *J* 18 and 8.5 Hz, 4-$CH_a$), 3.72 (1H, dd, *J* 18 and 10 Hz, 4-$CH_b$), 3.83 (1H, dd, *J* 3 and 6 Hz, 6-CH), 4.40 (1H, m, 5-CH), 5.35 and 5.56 (each 1H, d, *J* 14 Hz, C*H*$_2$Ar), *ca*. 5.38 (1H, m, C*H* Me), 6.41 (1H, dd, *J* 15.5 and 7.5 Hz, =C*H*CHO), 7.77 and 8.24 (each 2H, d, *J* 8.5 Hz, *Ar* $CH_2$), 8.20 (1H, s, OCHO), 8.23 (1H, dd, *J* 15.5 Hz, SC*H*=) and 9.54 (1H, d, *J* 7.5 Hz, CHO).

Preparation 28

p-Nitrobenzyl (5R,6S)-3-[(E)-3-hydroxyprop-1-enylthio]-6-[(R)-1-formyloxyethyl]-7-oxo-1-azabicyclo[3.2.0]hept-2-ene-2-carboxylate

The method of Preparation 16 was used to convert p-Nitrobenzyl (5R, 6S)-3-[(E)-3-oxoprop-1-enylthio]-6-[(R)-1-formyloxyethyl]-7-oxo-1-azabicyclo[3.2.0]hept-2-ene-2-carboxylate (256mg) into the title alcohol (117mg); $\lambda_{max}$ (EtOH) 324 and 262 nm; $\nu_{max}$ ($CH_2Cl_2$) 3610, 1785, 1725 and 1710 sh $cm^{-1}$; $\delta$($CDCl_3$) 1.45 (3H, d, J 6.5 Hz, MeCH), 1.68 (1H, br, OH), 3.11 (1H, dd, J 18 and 8.5 Hz, 4-$CH_a$), 3.34 (1H, dd, J 18 and 10 Hz, 4-$CH_b$), 3.37 (1H, dd, J 2.5 and 7 Hz, 6-CH), 4.23 (3H, m, 5-CH and $CH_2$OH), 5.26 and 5.50 (each 2H, d, J 14 Hz, $CH_2$Ar), 5.40 (1H, m, CHMe), 6.22 (1H, dt, J 15 and 4.5 Hz, =CH$CH_2$), 6.47 (1H, dt, J 15 and 1.5 Hz, SCH=), 7.65 and 8.23 (each 2H, d, J 9 Hz, Ar$CH_2$) and 8.05 (1H, s, OCHO).

Preparation 29

p-Nitrobenzyl (5R, 6S)-3-[(E)-3-azidoprop-1-enylthio]-6-[(R)-1-formyloxyethyl]-7-oxo-1-azabicyclo[3.2.0]hept-2-ene-2-carboxylate

p-Nitrobenzyl (5R, 6S)-3-[(E)-3-hydroxyprop-1-enylthio]-6-[(R)-1-formyloxyethyl]-7-oxo-1-azabicyclo [3.2.0]hept-2-ene-2-carboxylate (105mg) was reacted with hydrazoic acid under the conditions of Preparation 17 to afford the title azide (73mg); $\lambda_{max}$ (EtOH) 323 and 262 nm; $\nu_{max}$ ($CH_2Cl_2$) 2110, 1790, 1725 and 1705 sh $cm^{-1}$; $\delta$($CDCl_3$) 1.46 (3H, d, J 6.5 Hz, MeCH), 3.12 (1H, dd, J 8.5 and 18 Hz, 4-$CH_a$), 3.34 (1H, dd, J 10 and 18 Hz, 4-$CH_b$), 3.39 (1H, dd, J 3 and 7.5 Hz, 6-CH), 3.91 (2H, dd, J 1 and 6 Hz, $CH_2N_3$), 4.24 (1H, m, 5-CH), 5.27 and 5.51 (each 1H, d, J 13.5 Hz, $CH_2Ar$), 5.41 (1H, m, CHMe), 6.10 (1H, dt, J 6 and 15 Hz, =$CHCH_2$), 6.50 (1H, dt, J 1.5 and 15 Hz, SCH=), 8.06 (1H, s, OCHO), 7.65 and 8.23 (each 2H, d, J 9 Hz, $ArCH_2$).

Example 12

(5R, 6S)-3-[(E)-3-aminoprop-1-enylthio]-6-[(R)-1-hydroxyethyl]-7-oxo-1-azabicyclo[3.2.0]hept-2-ene-2-carboxylic acid

p-Nitrobenzyl (5R, 6S)-3-[(E)-3-azidoprop-1-enylthio]-6-[(R)-1-formyloxyethyl]-7-oxo-1-azabicyclo [3.2.0]hept-2-ene-2-carboxylate (40mg) was shaken with a prehydrogenated (30 min) mixture of 5% Pd-C (60mg) 1, 4-dioxan (20 ml), 0.05M pH 7 phosphate buffer solution (5ml) and water (5ml) for 2 h. The solution was filtered through Celite and the filtrate washed with three portions of ethyl acetate (30 ml). The aqueous solution was adjusted to pH 8.5 with 0.1N NaOH solution and warmed to 37°C. The pH was kept at 8.5 for 2 h. by the addition of further quantities of NaOH as necessary. After neutralising the solution to pH 7 with 0.1N HCl, it was concentrated in vacuo to small volume and loaded onto a column of Diaion HP 20. Elution with water, followed by 5% ethanol-water afforded the title amino-acid, $\lambda_{max}$ ($H_2O$) 303 nm.

## Preparation 30

### Ethyl (Z)-3-Triphenylmethylthiopropenoate

Triphenylmethyl mercaptan (1.0g) in DMF (10ml) was treated with aqueous 1N KOH (0.2ml). Ethyl propiolate (360mg) in DMF (4ml) was added. After 15 minutes the solution was neutralised by the addition of aqueous HCl. The solvents were removed by evaporation *in vacuo*. The residual gum was taken up in ethyl acetate (50ml) and washed with $H_2O$ (3 x 50ml), followed by brine (50ml). After drying ($MgSO_4$) the ethyl acetate was removed by evaporation *in vacuo*. Crystallisation of the residue from ethyl acetate/hexane gave pure ethyl (Z)-3-triphenylmethylthiopropenoate (650mg), m.p. 148 - 150°, $\nu_{max}$ ($CH_2Cl_2$) 1695, 1570, 1490, 1445, 1375 and 1355 $cm^{-1}$, δ(90 MHz; $CDCl_3$) 1.25 (3H, t, *J ca.* 6 Hz), 4.08 (2H, q, *J ca.* 6 Hz), 5.68 (1H, d, *J ca.* 10 Hz), 6.73 (1H, d, *J ca.* 10 Hz), 7.25 (15H, m) p.p.m. Found: C, 76.71; H, 6.07%. $C_{24}H_{22}O_2S$ requires C, 77.00; H, 5.88%.

Preparation 31

(Z)-3-Triphenylmethylthioprop-2-en-1-ol

Ethyl (Z)-3-triphenylmethylthiopropenoate (2.9g) in dry THF (150ml) was cooled in an ice bath and, under an argon atmosphere, treated with a 25% solution of diisobutylaluminium hydride in toluene (15ml) and the mixture was stirred for 2 hours. Water (10ml)/ethanol (10ml) were added, and after stirring for 3 minutes ethyl acetate (400ml) and water (200ml) and saturated brine solutions (100ml) were added. After filtration through Celite and separation of the layers the ethyl acetate layer was washed with brine. The combined aqueous layers were extracted with ethyl acetate, and this extract was washed with brine, and combined with the other ethyl acetate extract, dried ($MgSO_4$) and evaporated *in vacuo* to leave an oil. This was redissolved in ethyl acetate (*ca*. 30ml), treated with hexane (*ca*. 80ml) and placed in a refrigerator. This gave crystals of (Z)-3-triphenylmethylthioprop-2-en-1-ol (1.12g) m.p. 143 - 146°, $\nu_{max}$ ($CH_2Cl_2$) 3610, 1600, 1490, 1445, 1035, 1010 $cm^{-1}$, $\delta$(60 MHz, $CDCl_3$) 1.60 (1H, s, exch. $D_2O$), 4.23 (2H, d, *J ca*. 5.5 Hz), 5.35 (2H, m), 7.2 (15H, m) p.p.m., Found: C, 79.21; H, 6.05%. $C_{22}H_{20}O_5$ requires C, 79.52; H, 6.02%.

Preparation 32

(*Z*)-3-Triphenylmethylthioprop-2-en-1-ylazide

(*Z*)-3-Triphenylmethylthioprop-2-en-1-ol (1.06g) in THF (50ml) was treated with triphenylphosphine (1.67g), followed by hydrazoic acid in toluene (1.7M, 7.5ml). The mixture was cooled to $0^{o}$ and treated with diisopropyl azodicarboxylate (1.23ml; 1.29g) in THF (5ml). After 30 minutes ethyl acetate (100ml), aqueous $NaHCO_3$ (50ml) and brine (50ml) were added. After separation the aqueous layer was extracted with ethyl acetate. The combined ethyl acetate layers were washed with brine, dried ($MgSO_4$) and evaporated *in vacuo*. The product was chromatographed on silica gel (4.0 x 18cm), loading in toluene (30ml), and eluting with ethyl acetate/hexane 5 : 95. Early fractions contained the product, evaporation *in vacuo* and addition of hexane gave crystals of the title compound (556mg), m.p. 94 - $95^{o}$; $\nu_{max}$ ($CH_2Cl_2$) 2110, 1600, 1495 and 1445 $cm^{-1}$, $\delta$($CDCl_3$, 90 MHz), 3.92 (2H, d, *J* *ca*. 7 Hz), 5.50 (1H, dt, *J* 6.6 and 9.5 Hz), 5.94 (1H, d, *J* *ca*. 9 Hz), 7.25 (15H, broad s). Found: C, 74.30; H, 5.58; N, 11.37; S, 8.93%. $C_{22}H_{19}N_3S$ requires C, 73.95; H, 5.32; N, 11.76; S, 8.96%.

Evaporation of the mother liquoursgave a further crop of crystals of the title compound (493mg).

Preparation 33

(Z)-3-p-Nitrobenzyloxycarbonylamino-1-triphenylmethylthioprop-1-ene

METHOD A

(Z)-3-Triphenylmethylthioprop-2-en-1-ylazide (980mg) in toluene (5ml) was treated with triphenylphosphine (791mg) in toluene (7ml) and the mixture was heated at 70° under an atmosphere of argon. When evolution of nitrogen had ceased (ca. 20 min) the solution was cooled in an ice bath and treated with p-nitrobenzyl chloroformate (709mg). Immediately a gummy solid precipitated. After 20 min ethyl acetate (100ml) and aqueous $NaHCO_3$ (100ml) were added and the mixture was shaken until all the gummy solid had dissolved. The ethyl acetate layer was washed with brine, dried and evaporated in vacuo. The residue was chromatographed on silica gel (4 x 18cm) eluting with ethyl acetate/hexane mixtures; 1 : 9 (400ml), 2 : 8 (500ml), 3 : 7. Eventually the desired urethane was eluted. Evaporation in vacuo of the fractions containing this material gave the title compound as a gum (950mg). Crystallisation from ether gave (Z)-3-p-nitrobenzyloxycarbonylamino-1-triphenylmethylprop-1-ene, m.p. 139 - 140°, $\nu_{max}$ ($CH_2Cl_2$) 3430, 1725, 1605, 1510, 1350, 1225 $cm^{-1}$, δ($CDCl_3$. 60 MHz) 3.90 (2H, t, J ca. 6 Hz), 4.85 (1H, broad s), 5.16 (2H, s), 5.35 - 5.65 (1H, m), 5.85 (1H, d, J ca. 9 Hz), 7.25 (15H, m), 7.45 (2H, d, J ca. 9 Hz), 8.15 (2H, d, J ca. 9 Hz) p.p.m., Found: C, 70.60; H, 5.40; N, 5.52; S, 6.03%. $C_{30}H_{26}N_2O_4S$ requires C, 70.59; H, 5.10; N, 5.49; S, 6.29%.

METHOD B

(Z)-3-Triphenylmethylthioprop-2-en-1-ylazide (110mg) in dry THF (3ml) was treated with triphenylphosphine (81mg) in dry THF (1ml). The solution was warmed to reflux for 15 min and then allowed to stand at ambient temperature for 1 hr. Water (0.008ml) was then added, and after 1 hr more water (0.024ml) was added and the mixture was heated under reflux for 30 min. The solvents were removed by evaporation *in vacuo* to give the crude amino compound. This was taken up in dichloromethane and treated with triethylamine (38mg), followed by p-nitrobenzyl chloroformate (85mg). After 30 min the solution was washed with water, and then with brine. After drying ($MgSO_4$) the $CH_2Cl_2$ was evaporated *in vacuo* and chromatographed on silica gel eluting with ethyl acetate/hexane mixture to give the title compound (85mg).

Preparation 34

Silver (Z)-3-p-Nitrobenzyloxycarbonylamino-prop-1-en-1-ylthiolate

(Z)-3-p-Nitrobenzyloxycarbonylamino-1-triphenylmethylthioprop-1-ene (340mg) in warm methanol (30ml) was heated at $40^o$ under argon and treated with silver nitrate (113mg) followed by pyridine (52mg). The mixture was stirred at $40^o$ for 1.5 h, then allowed to cool to ambient and stirred for a further 0.5 h. The solid was isolated by centrifugation, washed with methanol (2 x 30ml), followed by ether (2 x 30ml). The resultant solid was dried in a vacuum desiccator to give the title silver salt (221mg), $\nu_{max}$ (KBr) 3400, 1700, 1605, 1515, 1385, 1345 and 1250 $cm^{-1}$.

Preparation 35

(Z)-1-Acetylthio-3-p-Nitrobenzyloxycarbonylaminoprop-1-ene

Silver (Z)-3-p-nitrobenzyloxycarbonylaminoprop-1-ene (877mg) was dissolved in warm DMF (10ml) and acetonitrile (10ml) was then added and, after cooling to ambient temperature, pyridine (0.3ml) followed by acetyl chloride (0.2ml) was added. After 10 minutes the mixture was filtered through Celite and the solvents removed by evaporation *in vacuo*. Ethyl acetate and water were added, the layers separated and the ethyl acetate layer was washed with water (2x) followed by brine, then dried ($MgSO_4$) and evaporated *in vacuo*. Chromatography on silica gel (0.040 - 0.063 mm/230 - 400 mesh ASTM) (3.0 x 12 cm), eluting with ethyl acetate/hexane mixtures; 2 : 8 (200ml), 3 : 7 (200 ml), 4 : 6 gave the desired product.

Crystallisation from ethyl acetate/hexane gave (Z)-1-acetylthio-3-p-nitrobenzyloxycarbonylaminoprop-1-ene (510mg), m.p. 107 - 109°, $\nu_{max}$ ($CH_2Cl_2$) 3425, 1720, 1700, 1600, 1510, 1345, 1220, 1100 $cm^{-1}$, $\delta$($CDCl_3$, 60 MHz) 2.39 (3H, s), 3.83 (2H, approx t, *J ca.* 6 Hz), 5.15 (2H, centre of ABq), 5.5 - 6.0 (1H, m), 6.60 (1H, d, *J ca.* 9 Hz), 7.45 (2H, d, *J ca.* 9 Hz), 8.10 (2H, d, *J ca.* 9 Hz), Found: C, 50.16; H, 4.55; N, 8.96; S, 10.45%. $C_{13}H_{14}N_2O_5S$ requires C, 50.32; H, 4.52; N, 9.03; S, 10.32%.

Preparation 36

p-Nitrobenzyl (5RS, 6SR)-6-[(1RS)-1-p-Nitrobenzyloxycarbonyloxyethyl]-3-[(Z)-3-p-nitrobenzyloxycarbonylaminoprop-1-en-1-ylthio]-7-oxo-1-azabicyclo[3.2.0]hept-2-ene-2-carboxylate

(Z)-1-Acetylthio-3-p-nitrobenzyloxycarbonylaminoprop-1-ene (211mg) in dioxan (10ml) was treated with 1M NaOH (aq.) (1.36ml). The solution was allowed to stir at ambient temperature for 20 min. Water (5ml) was added and the mixture was evaporated *in vacuo* to *ca*. 5ml. A further 5ml of water was added and the mixture was stirred and cooled in an ice-bath. p-Nitrobenzyl (5RS, 6SR)-6-[(1RS)-1-p-nitrobenzyloxycarbonyloxyethyl]-3-(2-pyrimidinylsulphinyl)-7-oxo-1-azabicyclo[3.2.0]hept-2-ene-2-carboxylate (393mg) and benzyldimethyl-n-hexadecylammonium chloride (25mg) in dichloromethane (10ml) were added and the mixture was stirred rapidly for 4 minutes. The layers were separated and the dichloromethane was washed with water, then with brine, then dried ($MgSO_4$) and evaporated *in vacuo*. The residue was chromatographed on silica gel (3.0 x 16cm) eluting with ethyl acetate/hexane mixtures; 1 : 1 (250ml), 6 : 4 (250ml), 65 : 35 (250ml), 7 : 3. Fractions containing the title compound were combined and evaporated *in vacuo* to give the title compound (320mg) $\nu_{max}$ ($CH_2Cl_2$) 3440, 1775, 1745 (sh), 1730, 1700 (sh), 1525, 1350 $cm^{-1}$, δ($CDCl_3$, 250 MHz), 1.49 (3H, d, *J*, 6.3 Hz), 3.08 (1H, dd, *J* 17.9 and 8.5 Hz), 3.27 (1H, dd, *J* 18.1 and 9.9 Hz), 3.39 (1H, dd, *J* 7.9 and 2.7 Hz), 4.01 (2H, t, *J* 6.2 Hz), 4.23 (1H, m), 5.03 (1H, broad, NH), *ca*. 5.15 (1H, m), 5.19 - 5.3 (5H, m), 5.50 (1H, d, *J* 13.8 Hz), 5.98 (1H, m), 6.33 (1H, d, *J* 9.5 Hz), 7.50 (2H, d, *J* 8.5 Hz), 7.59 (2H, d, *J* 8.8 Hz), 7.65 (2H, d, *J* 8.7 Hz), 8.2 - 8.3 (6H, m) p.p.m.

Preparation 37

Silver (Z)-3-Azidoprop-1-en-1-ylthiolate

(Z)-3-Azido-1-triphenylmethylthioprop-1-ene (714mg) in warm methanol (15ml) was heated at $40^{o}$ under argon and pyridine (166mg) followed by finely ground silver nitrate (340mg) were added. The mixture was stirred at room temperature for 2 hr. The brown precipitate was collected by filtration and was washed with methanol (6 x 10ml) followed by ether (2 x 30ml). The resultant solid was dried in a vacuum desiccator to give the title silver salt (351mg) which was used immediately in Preparation 38.

Preparation 38

p-Nitrobenzyl (5RS, 6SR)-6-[(1RS)-1-p-nitrobenzyloxycarbonyloxyethyl]-3-[(Z)-3-azidoprop-1-en-1-ylthio]-7-oxo-1-azabicyclo[3.2.0]hept-2-ene-2-carboxylate

p-Nitrobenzyl (5RS, 6SR)-6-[(1RS)-1-p-nitrobenzyloxycarbonyloxyethyl]-3-(2-pyrimidinylsulphinyl)-7-oxo-1-azabicyclo[3.2.0]hept-2-ene-2-carboxylate (61mg) was dissolved in acetonitrile (5ml) and the mixture was cooled in an ice-bath. Sodium iodide (143mg) followed by silver (Z)-3-azidoprop-1-en-1-ylthiolate (100mg) were added and the mixture stirred for 10 minutes. The solid was removed by centrifugation and the precipitate washed with dichloromethane (2 x 10ml). The dichloromethane and acetonitrile solutions were combined and evaporated under reduced pressure to give a yellow solid. This solid was suspended in dichloromethane (10ml) and centrifuged. The procedure was repeated (x3) and the dichloromethane layers which were removed on each occasion were combined and evaporated *in vacuo* to give an oil which was chromatographed on silica gel eluting with ethyl acetate/hexane solvent mixture (1 : 1). The fractions containing the title compound were combined and evaporated *in vacuo* to give the title compound (16mg); $\lambda_{max}$ (EtOH) 322.3 nm and 260.5 nm; $\nu_{max}$ ($CH_2Cl_2$) 2930, 2100, 1785, 1750, 1705, 1610, 1560, 1525, 1380, 1350, 1335, 1245, 1200, 1140, 1035 and 850 $cm^{-1}$; $\delta$($CDCl_3$, 250 MHz) 1.49 (3H, d, *J* 6.4 Hz), 3.10 (1H, dd, *J* 18.1 and 8.4 Hz), 3.31 (1H, dd, *J* 18.0 and 9.8 Hz), 3.41 (1H, dd, *J* 7.9 and 2.9 Hz), 4.02 (2H, m), 4.25 (1H, m), *ca*.5.16 (1H, m), 5.25 (2H,s), 5.27 (1H, d, *J* 13.7 Hz), 5.51 (1H, d, *J* 13.7 Hz), 6.01 (1H, dt, *J* 7.0 and 9.4 Hz), 6.50 (1H, d, *J* 9.4 Hz), 7.54 (2H, d, *J* 8.8 Hz), 7.64 (2H,d,*J* 8.7 Hz), 8.22 (2H, d,*J* 8.7 Hz) and 8.23 (2H, d, *J* 8.8 Hz).

Example 13

(5RS, 6SR)-3-[(Z)-3-Aminoprop-1-enylthio]-6-[(1RS)-1-hydroxyethyl]-7-oxo-1-azabicyclo[3.2.0]hept-2-ene-2-carboxylic acid

METHOD A

5% Pd/C catalyst (300mg) in dioxan (80ml)/water (20ml)/0.05M pH 7 potassium phosphate buffer (20ml) was prehydrogenated for 20 min. p-Nitrobenzyl (5RS, 6SR)-6-[(1RS)-1-p-nitrobenzyloxycarbonyloxyethyl]-3-[(Z)-3-p-nitrobenzyloxycarbonylaminoprop-1-en-1-ylthio]-7-oxo-1-azabicyclo[3.2.0]hept-2-ene-2-carboxylate (200mg) was added and the mixture was hydrogenated at atmospheric pressure for 1.5 hours. The catalyst was removed by filtration through Celite, and the Celite was washed with water (40ml). The solution was evaporated in vacuo to ca. 100 ml and washed with ethylacetate (2x). The aqueous layer was evaporated in vacuo to ca. 50ml. Chromatography of half of this solution on Diaion HP-20 (2.3 x 15cm) eluting with water gave the purified title compound in later fractions. These were combined and freeze dried to give the title compound.

METHOD B

5% Pd/C catalyst (7.0mg) in dioxan (2ml)/water (0.5ml)/0.05M pH 7.0 potassium phosphate buffer (1ml) was prehydrogenated for 45 minutes. p-Nitrobenzyl (5RS, 6SR)-6-[(1RS)-1-p-nitrobenzyloxycarbonyloxyethyl]-3-[(Z)-3-azidoprop-1-en-1-ylthio]-7-oxo-1-azabicyclo[3.2.0]hept-2-ene-2-carboxylate (5mg) was added and the mixture was hydrogenated at atmospheric pressure for 1.5 hours. The catalyst was removed by filtration through Celite and the Celite was washed with water

(20ml). The solution was evaporated *in vacuo* to *ca*. 10ml and washed with ethyl acetate (3x). The aqueous layer was evaporated *in vacuo* to 3ml to give a solution of the title compound, $\lambda_{max}$ ($H_2O$) 297.7 nm.

## Preparation 39

### Methyl (Z)-3-triphenylmethylthiobut-2-enoate

Triphenylmethylmercaptan (28.15gm) and methyl tetrolate (10gm) were dissolved in N,N-dimethylformamide (125ml). 1M Potassium hydroxide solution (1ml) was added and the resulting solution was stirred at room temperature for 1 hour. The solution was then neutralised by the addition of 1M hydrochloric acid solution (1ml) and evaporated at reduced pressure. The residual oil was partitioned between ethyl acetate and water. The organic layer was separated and washed with water (x3), brine and dried ($MgSO_4$). Evaporation of the organic solution at reduced pressure gave an orange oil, which was chromatographed over silica gel. Elution with a gradient of hexane to 10% ethyl acetate/hexane afforded the title compound as a white solid (15gm), $\nu_{max}$ ($CHCl_3$) 1688, 1570$cm^{-1}$; $\delta_H$ ($CDCl_3$) 1.48 (3H, s, MeC=C), 3.62 (3H, s, $CO_2Me$), 5.55 (3H, s, HC=C), 7.20 (15H, m, aromatic H).

Preparation 40

(Z)-3-Triphenylmethylthiobut-2-en-1-ol

The title compound was prepared from methyl (Z)-3-triphenylmethylthiobut-2-enoate by the procedure described in preparation 31, $\nu_{max}$ ($CHCl_3$) 3550cm$^{-1}$; $\delta_H$ ($CDCl_3$) 1.28 (1H, broad s, OH), 1.62 (3H, s, MeC=C), 3.90 (2H, d, $CH_2OH$), 5.62 (1H, t, HC=C), 7.0 - 7.5 (15H, m, aromatic H).

## Preparation 41

## (Z)-3-Triphenylmethylthiobut-2-en-1-yl azide and (E)-3-Triphenylmethylthiobut-2-en-1-yl azide

The inseparable mixture (2:1) of (Z)-3-triphenylmethylthiobut-2-en-1-yl azide and (E)-3-triphenylmethylthiobut-2-en-1-yl azide was prepared from (Z)-3-triphenylmethylthiobut-2-en-1-ol by the procedure described in preparation 32, $\nu_{max}$ ($CHCl_3$) 2100cm$^{-1}$, $\delta_H$ ($CDCl_3$) 1.58 (s, major) and 1.68 (s, minor) (3H, MeC=C), 3.31 (d, minor) and 3.60 (d, major) (2H, $CH_2N_3$), 5.0 (t, minor) and 5.48 (t, major) (1H, HC=C), 6.9 - 7.5 (15H, m, aromatic H).

## Preparation 42

**(Z)-1-p-Nitrobenzyloxycarbonylamino-3-triphenylmethylthiobut-2-ene and (E)-1-p-Nitrobenzyloxycarbonylamino-3-triphenylmethylthiobut-2-ene**

The title compounds were prepared from the mixture of (Z)-3-triphenylmethylthiobut-2-en-1-yl azide and (E)-3-triphenylmethylthiobut-2-en-1-yl azide by the procedure described in preparation 33, method A. They were separated by silica gel column chromatography, eluting with a gradient of 10 to 25% ethyl acetate/hexane. The first eluted component was the major product, (Z)-1-p-nitrobenzyloxycarbonylamino-3-triphenylmethylthiobut-2-ene, $\nu_{max}$ ($CHCl_3$) 3450, 1720, 1603cm$^{-1}$; $\delta_H$ ($CDCl_3$) 1.61 (3H, s, MeC=C), 3.66 (2H, t, $CH_2N$), 4.20 (1H, broad res., NH), 5.11 (2H, s, $CH_2Ar$), 5.58 (1H, t, HC=C), 7.1 - 7.6 (m, aromatic protons), 8.18 (2H, d, aromatic protons). The second eluted component was the minor isomer, (E)-1-p-nitrobenzyloxycarbonylamino-3-triphenylmethylthiobut-2-ene, $\nu_{max}$ ($CH_2Cl_2$) 3440, 1722, 1602cm$^{-1}$; $\delta_H$ ($CDCl_3$) 1.62 (3H, s, MeC=C), 3.48 (2H, t, $CH_2N$), 4.03 (1H, broad res., NH), 4.8 - 5.3 (3H, m + s, $CH_2Ar$ + HC=C), 7.0 - 7.6 (m, aromatic protons), 8.18 (2H, d, aromatic protons).

Preparation 43

Silver salt of (E)-1-p-nitrobenzyloxycarbonylamino-3-mercaptobut-2-ene

(E)-1-p-nitrobenzyloxycarbonylamino-3-triphenylmethylthiobut-2-ene (500mg) was dissolved in dry methanol (50ml) by heating to reflux. Pyridine (0.092ml; 0.090gm), followed by powdered silver nitrate (0.195gm) was added and the mixture was stirred at ambient temperature, under an argon atmosphere, for 3 hours. The solid was isolated by centrifugation and washed with methanol (x2) and diethyl ether (x2). The resulting pale yellow solid was dried under vacuum and stored under argon , in the dark.

Preparation 44

(Z)-1-p-Nitrobenzyloxycarbonylamino-3-formylthiobut-2-ene

(Z)-1-p-Nitrobenzyloxycarbonylamino-3-triphenylmethylthiobut-2-ene (0.500gm) was dissolved in dry acetonitrile (50ml) and stirred at room temperature for 3 hours, under an argon atmosphere, with pyridine (0.077ml), methanol (0.031ml) and powdered silver nitrate (0.162gm). To this mixture was then added acetic-formic anhydride (0.839gm), 4-dimethylaminopyridine (0.105gm) and triethylamine hydrochloride (1.312gm) at ice-bath temperature. The suspension was stirred for an additional 30 minutes at room temperature, and then filtered through Celite. The filtrate was evaporated at reduced pressure and the residue was partitioned between ethyl acetate and 5% citric acid solution. The organic layer was washed with saturated sodium chloride solution, saturated sodium bicarbonate solution, saturated sodium chloride solution again and dried over anhydrous magnesium sulphate. Evaporation of the solvent gave the crude product which was purified by rapid silica gel column chromatography (40gm). Elution with 25% ethyl acetate/hexane gave the title compound as a white solid (0.140gm), $\nu_{max}$ ($CH_2Cl_2$) 3440, 1725, 1679, 1608$cm^{-1}$; $\delta_H$ ($CDCl_3$) 2.11 (3H, s, $C\underline{H}_3C{=}C$), 3.87 (2H, t, $CH_2N$), 5.20 (3H, s + broad res., $C\underline{H}_2Ar$ + NH), 6.10 (1H, broad t, $\underline{H}C{=}C$), 7.41 (2H, d, aromatic protons), 8.16 (2H, d, aromatic protons), 10.00 (1H, s, $C\underline{H}O$).

Preparation 45

p-Nitrobenzyl (5RS, 6SR)-3-[(E)-3-p-nitrobenzyloxycarbonylamino-1-methylprop-1-enylthio]-6-[(RS)-1-p-nitrobenzyloxycarbonyloxyethyl]-7-oxo-1-azabicyclo[3.2.0]hept-2-ene-2-carboxylate

Reaction of the silver salt of (E)-1-p-nitrobenzyloxycarbonylamino-3-mercaptobut-2-ene with p-nitrobenzyl (5RS, 6SR)-3-(2-pyrimidinylsulphinyl)-6-[(RS)-1-p-nitrobenzyloxycarbonyloxyethyl]-7-oxo-1-azabicyclo[3.2.0]hept-2-ene-2-carboxylate by the procedure outlined in preparation 38 afforded the title compound as an oil, $\nu_{max}$ ($CHCl_3$) 3450, 1782, 1722, 1608cm$^{-1}$; $\delta_H$ ($CDCl_3$) 1.48 (3H, d, J 6.5Hz, $CH_3CH$), 2.08 (3H, s, $CH_3C{=}C$), 3.01 (1H, dd, 4 - $H_a$), 3.13 (1H, dd, 4 - $H_b$), 3.36 (1H, dd, J 2.5 and 8.0 Hz, 6 - H), 3.90 (2H, broad res., $CH_2N$), 4.18 (1H, dt, 5 - H), 5.1 - 5.55 (8H, m, 3 x $CH_2Ar$ + 8 - H + NH), 6.05 (1H, broad t, HC=C), 7.45 - 7.7 (m, aromatic protons), 8.21 (d, aromatic protons).

Example 14

(5RS, 6SR)-3-[(E)-3-amino-1-methylprop-1-enylthio]-6-[(RS)-1-hydroxyethyl]-7-oxo-1-azabicyclo[3.2.0]hept-2-ene-2-carboxylic acid

The title compound was prepared as an aqueous solution by hydrogenolysis of p-nitrobenzyl (5RS, 6SR)-3-[(E)-3-p-nitrobenzyloxycarbonylamino-1-methylprop-1-enylthio]-6-[(RS)-1-p-nitrobenzyloxycarbonyloxyethyl]-7-oxo-1-azabicyclo[3.2.0]hept-2-ene-2-carboxylate, following the procedure outlined in example 13, $\nu_{max}$ ($H_2O$) 299nm. In this case the hydrogenolysis time was extended to 3 hours.

## Preparation 46

p-Nitrobenzyl (5R, 6S)-3-[(Z)-3-p-nitrobenzyloxycarbonyl-amino-1-methylprop-1-enylthio]-6-[(R)-1-hydroxyethyl]-7-oxo-1-azabicyclo[3.2.0]hept-2-ene-2-carboxylate

A. (Z)-1-p-Nitrobenzyloxycarbonylamino-3-formylthio-but-2-ene (0.570gm) was dissolved in 1, 4-dioxan (50ml) and stirred at room temperature for 30 minutes with 1N sodium hydroxide solution (3.68ml). The 1, 4-dioxan was then removed by evaporation at reduced pressure. The resulting solution was diluted with water (25ml).

B. p-Nitrobenzyl (5R, 6S)-3-[(E)-2-acetamidovinylthio]-6-[(R)-1-hydroxyethyl]-7-oxo-1-azabicyclo[3.2.0]hept-2-ene-2-carboxylate (0.512gm) was converted to its sulphoxide by established procedures. This was then dissolved in dichloromethane (100ml) and vigorously stirred for 15 minutes with the aqueous solution obtained from part A, in the presence of cetyl dimethylbenzylammonium chloride (0.051gm). The two layers were separated and the organic layer washed with water, saturated sodium chloride solution and dried ($MgSO_4$). Removal of the solvent at reduced pressure afforded the crude product, which was purified by silica gel column chromatography (50gm). Elution with a gradient of 25 - 75% ethyl acetate/hexane gave the title compound as a pale yellow foam (0.101gm), $\nu_{max}$ ($CH_2Cl_2$) 3600, 3450, 1780, 1725, 1608cm$^{-1}$; $\lambda_{max}$ (EtOH) 317, 265nm; $\delta_H$ ($CDCl_3$) 1.35 (3H, d, J 6.5 Hz, C$\underline{H}_3$CH), 1.82 (1H, d, O$\underline{H}$), 2.10 (3H, d, J 1.0 Hz, C$\underline{H}_3$C=C), 3.00 (1H, dd, 4 - $H_a$), 3.13 (1H, dd, 4 - $H_b$), 3.20 (1H, broad d, 6 - H), 4.03 (2H, broad res., C$\underline{H}_2$N), 4.22 (2H, m, 5 -H + 8 - H), 5.03 (1H, broad t, N$\underline{H}$), 5.20 (2H, s, C$\underline{H}_2$Ar), 5.25 (d) and 5.52 (d) (2H, $CH_2$Ar),

6.04 (1H, broad t, HC=C), 7.50 (2H, d, aromatic), 7.67 (2H, d, aromatic), 8.21 (4H, d, aromatic).

Example 15

(5R, 6S)-3-[(Z)-3-amino-1-methylprop-1-enylthio]-6-[(R)-1-hydroxyethyl]-7-oxo-1-azabicyclo[3.2.0]hept-2-ene-2-carboxylic acid

Hydrogenolysis of p-nitrobenzyl (5R, 6S)-3-[(Z)-3-p-nitrobenzyloxycarbonylamino-1-methylprop-1-enylthio]-6-[(R)-1-hydroxyethyl]-7-oxo-1-azabicyclo[3.2.0]hept-2-ene-2-carboxylate, by the procedure described in example 13, gave the title compound, also described in example 10, as a white fluffy solid after Biogel P-2 column chromatography and lyophilisation, $\lambda_{max}$ ($H_2O$) 299nm, $\nu_{max}$ (KBr) 3250, 1760cm$^{-1}$. The hydrogenolysis time in this case was extended to 3 hours.

Preparation 47

p-Nitrobenzyl (5R, 6R)-3-[(E)-3-hydroxy-1-methylprop-1-enylthio]-6-[(S)-1-trimethylsilyloxyethyl]-7-oxo-1-azabicyclo[3.2.0]hept-2-ene-2-carboxylate

When Preparation 16 was performed on a sufficiently large scale, small quantities of the title compound could be isolated in pure form (12% yield) on continued elution of the silica gel column, $\nu_{max}$ ($CHCl_3$) 3420, 1780, 1705, 1611 $cm^{-1}$; $\lambda_{max}$ (EtOH) 317, 265nm; $\delta_H$ ($CDCl_3$) 1.30 (3H, d, J 6 Hz, $CH_3CH$), 2.02 (3H, s, $CH_3C{=}C$), 2.86 (1H, dd, 4 - $H_a$), 3.4 - 3.9 (2H, m, 4 - $H_a$ + 6 - H), 4.1 - 4.4 (4H, m, $CH_2O$ + 5 - H + 8 - H), 5.21 (d) + 5.48 (d) (2H, $CH_2Ar$), 6.21 (1H, t, $HC{=}C$), 7.64 (2H, d, aromatic protons), 8.18 (2H, d, aromatic protons).

Preparation 48

p-Nitrobenzyl (5R, 6R)-3-[(E)-3-azido-1-methylprop-1-enylthio]-6-[(S)-1-trimethylsilyloxyethyl]-7-oxo-1-azabicyclo[3.2.0]hept-2-ene-2-carboxylate

The title compound was prepared from p-nitrobenzyl (5R, 6R)-3-[(E)-3-hydroxy-1-methylprop-1-enylthio]-6-[(S)-1-trimethylsilyloxyethyl]-7-oxo-1-azabicyclo [3.2.0]hept-2-ene-2-carboxylate by the procedure outlined in preparation 17, $\lambda_{max}$ (EtOH) 317, 264nm; $\nu_{max}$ ($CHCl_3$) 2100, 1775, 1721, 1605cm$^{-1}$; $\delta_H$ ($CDCl_3$) 0.12 (9H, s, $Me_3Si$), 1.33 (3H, d, $CH_3CH$), 2.12 (3H, d, J ca. 1 Hz, $CH_3C=C$), 2.92 (1H, dd, 4 - $H_a$), 3.5 - 3.75 (2H, m, 4 - $H_b$ + 6 - H), 3.89 (2H, d, $CH_2N_3$), 4.2 - 4.36 (2H, m, 5 - H + 8 - H), 5.28 (d) + 5.51 (d) (2H, $CH_2Ar$), 6.18 (1H, dt, HC=C), 7.66 (2H, d, aromatic protons), 8.22 (2H, d, aromatic protons).

Example 16

(5R, 6R)-3-[(E)-3-amino-1-methylprop-1-enylthio]-6-[(S)-1-hydroxyethyl]-7-oxo-1-azabicyclo[3.2.0]hept-2-ene-2-carboxylate

The title compound was prepared as an aqueous solution by hydrogenolysis of p-nitrobenzyl (5R, 6R)-3-[(E)-3-azido-1-methylprop-1-enylthio]-6-[(S)-1-trimethylsilyloxyethyl]-7-oxo-1-azabicyclo[3.2.0]hept-2-ene-2-carboxylate, following the procedure outlined in example 1, $\lambda_{max}$ ($H_2O$) 298nm. In this case, the hydrogenolysis time was extended to 3 hours.

Antibacterial Activity of Aminopropenyl Derivatives

| | MIC+ µg/ml | | | |
|---|---|---|---|---|
| Compound of Example | R + E coli | S aureus Russell | S typhimurium CT10 | P aeruginosa A |
| 1 | 0.4 | 0.1 | 0.2 | 6.2 |
| 2 | 0.3 | 0.08 | 0.6 | 25 |
| *13 | 0.4 | 0.08 | 0.6 | 25 |
| 8 | 1.6 | <0.1 | <0.1 | 25 |
| 10 | 0.2 | 0.1 | 0.2 | 25 |
| 11 | <0.1 | <0.1 | 0.4 | 12.5 |
| 12 | 0.2 | <0.1 | 0.2 | 12.5 |
| 16 | 1.6 | <0.1 | 0.2 | 25 |
| *14 | 0.4 | <0.1 | 0.4 | 25 |

*(Racemic - not corrected for presence of inactive enantiomer).

+(Microtitre using Nutrient broth).

## Stability in Kidney Preparations

| Example | % left after 60 minutes | |
|---|---|---|
| | Pig Kidney Acetone Powder (100 mg/ml solubilised in 5% Triton X-100) | Human Kidney Sonicate (15% solubilised in 5% Triton X-100) |
| 1 | 39 | 40 |
| *13 | 40 | 39 |
| 8 | 78 | 51 |
| *10 | 77 | 56 |
| 11 | 6 | 7 |
| 12 | 8 | 5 |
| 16 | 38 | 13 |
| *14 | 36 | 23 |

*(These are racemic compounds: Figures are based on stability of bio-active enantiomer).

CLAIMS

1 A compound of formula (I) or an amidine derivative thereof or a pharmaceutically acceptable salt or ester thereof:

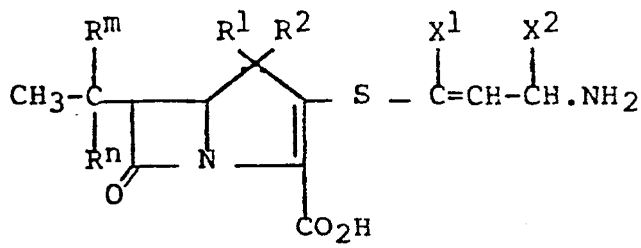

(I)

wherein $R^m$ is hydrogen or a group -OR, $R^n$ is hydrogen or methyl, R is hydrogen, -CHO or $-SO_3H$ or a pharmaceutically acceptable salt thereof, $R^1$ and $R^2$ are independently hydrogen, $C_{1-6}$ alkyl, or $R^1$ and $R^2$ are joined so as to form with the carbon to which they are attached a $C_{3-7}$ cycloalkyl ring, and $X^1$ and $X^2$ are independently hydrogen or optionally substituted hydrocarbon.

2 A compound as claimed in claim 1 wherein $X^1$ and $X^2$ independently represent hydrogen, $C_{1-6}$ alkyl or optionally substituted aryl.

3 A compound as claimed in either of claims 1 or 2 wherein $R^m$ represents a group -OR, and $R^n$ represents hydrogen.

4 A compound as claimed in any one of claims 1 - 3 wherein R is hydrogen or -CHO.

5 An amidine compound, as claimed in claim (I), of formula (II) or a pharmaceutically acceptable salt or ester thereof:

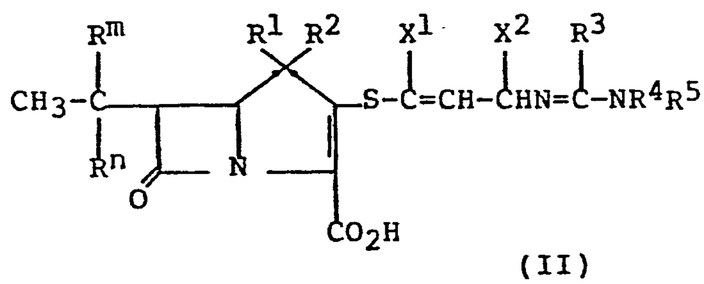

(II)

wherein $R^m$,$R^n$, $R^1$, $R^2$, $X^1$ and $X^2$ are as defined in claim 1 and $R^3$, $R^4$ and $R^5$ are independently selected from hydrogen or an optionally substituted hydrocarbon or heterocyclic group; or $R^4$ and $R^5$ together with the nitrogen to which they are attached form a 3-10 membered heterocyclic ring.

6 A compound as claimed in claim 5 wherein $R^3$, $R^4$ and $R^5$ are independently hydrogen or $C_{1-6}$ alkyl.

7 A compound selected from any of the following or a pharmaceutically acceptable salt or ester thereof:

(5R,6R)-3-[(Z)-3-aminoprop-1-enylthio]-6-[(S)-1-hydroxyethyl]-7-oxo-1-azabicyclo-[3.2.0]hept-2-ene-2-carboxylic acid;

A (5R,6S)-3-[(Z)-3-aminoprop-1-enylthio]-6-[(R)-1-hydroxyethyl]-7-oxo-1-azabicyclo[3.2.0]hept-2-ene-2-carboxylic acid;

(5R,6R)-3-(3-aminobut-1-en-1-ylthio)-6-[(S)-1-hydroxyethyl]-7-oxo-1-azabicyclo[3.2.0]hept-2-ene-2-carboxylic acid;

(5R,6S)-3-(3-aminobut-1-en-1-ylthio)-6-[(R)-1-formyloxyethyl]-7-oxo-1-azabicyclo[3.2.0]hept-2-ene-2-carboxylic acid;

(5R,6R)-3-[(Z)-3-acetimidoylaminoprop-1-enylthio]-6-[(S)-1-hydroxyethyl]-7-oxo-1-azabicyclo[3.2.0]hept-2-ene-2-carboxylic acid;

(5R,6R)-3-[(Z)-3-formimidoyl-aminoprop-1-enylthio] 6-[(S)-1-hydroxyethyl]-7-oxo-1-azabicyclo[3.2.0]hept-2-ene-2-carboxylic acid;

(5R,6S)-3-(3-aminobut-1-en-1-ylthio)-6-[(R)-1-hydroxyethyl]-7-oxo-1-azabicyclo[3.2.0]hept-2-ene-2-carboxylic acid;

(5R,6R)-3-[(Z)-3-amino-1-methylprop-1-enylthio]-6-[(S)-1-hydroxyethyl]-7-oxo-1-azabicyclo[3.2.0]hept-2-ene-2-carboxylic acid;

(5R,6R)-3-(3-amino-1-phenylprop-1-enylthio)-6-[(S)-1-hydroxyethyl]-7-oxo-1-azabicyclo[3.2.0]hept-2-ene-2-carboxylic acid;

(5R,6S)-3-[(Z)-3-amino-1-methylprop-1-enylthio]-6-[(R)-1-hydroxyethyl]-7-oxo-1-azabicyclo[3.2.0]hept-2-ene-2-carboxylic acid;

(5R,6R)-3-[(E)-3-aminoprop-1-enylthio]-6-[(S)-1-hydroxyethyl]-7-oxo-1-azabicyclo[3.2.0]hept-2-ene-2-carboxylic acid;

(5R,6S)-3-[(E)-3-aminoprop-1-enylthio]-6-[(R)-1-hydroxyethyl]-7-oxo-1-azabicyclo[3.2.0]hept-2-ene-2-carboxylic acid;

(5RS,6SR)-3-[(Z)-3-aminoprop-1-enylthio]-6-[(1RS)-1-hydroxyethyl]-7-oxo-1-azabicyclo[3.2.0]hept-2-ene-2-carboxylic acid;

(5RS,6SR)-3-[(E)-3-amino-1-methylprop-1-enylthio]-6-[(RS)-1-hydroxyethyl]-7-oxo-1-azabicyclo[3.2.0]hept-2-ene-2-carboxylic acid;

(5R,6R)-3-[(E)-3-amino-1-methylprop-1-enylthio]-6-[(S)-1-hydroxyethyl]-7-oxo-1-azabicyclo[3.2.0]hept-2-ene-2-carboxylic acid.

8 A process for the preparation of a compound as claimed in claim (I) which process comprises either

A) reacting a compound of formula (III):

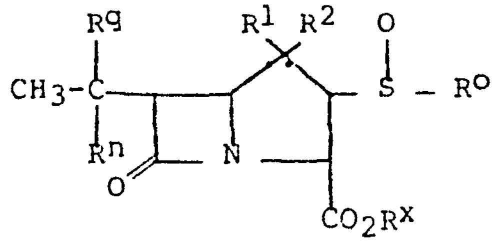

(III)

wherein $R^1$ and $R^2$ are as defined in claim 1, $R^q$ is hydrogen or a group $-OR^p$, where $R^p$ is a group R as defined in claim 1 or a protected derivative thereof, $R^x$ is a carboxy protecting group and $R^o$ is an optionally substituted hydrocarbon or heterocyclic group;

with a compound of formula (IV) or a salt or an amidine derivative thereof:

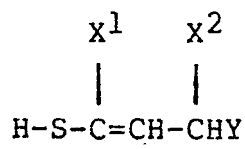

(IV)

where Y is amino, protected amino, or a precursor thereof;

or,

B) the elimination of the elements of $R^aR^bR^cP=0$ from an ester of a compound of the formula (V) or an amidine derivative thereof:

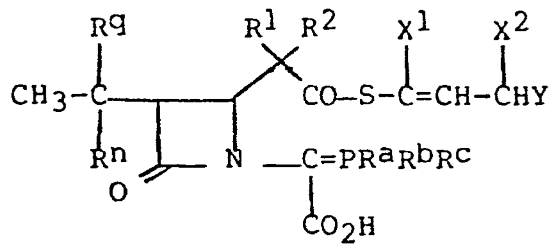

(V)

wherein $R^n$, $X^1$, $X^2$, $R^1$ and $R^2$ are as defined in claim 1, $R^q$ is as defined with respect to formula (III), Y is as defined with respect to formula (IV) and $R^a$, $R^b$ and $R^c$ are independently phenyl or methoxyphenyl;

or,

C) the reduction of a compound of formula (X):

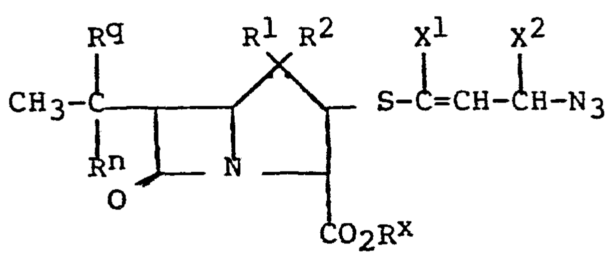

(X)

wherein $R^1$, $R^2$ and $R^n$ are as defined in claim 1 and $R^q$ and $R^x$ are as defined with respect to formula (III); and thereafter if necessary one or more of the following steps:

4 a) removing any protecting and/or blocking groups;

b) treating with dilute alkali;

c) reacting a compound of formula (I) or a protected derivative thereof with either a compound of formula (VIII) or (IX):

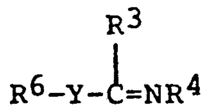

(VIII)

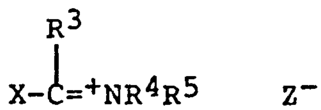

(IX)

wherein $R^3$, $R^4$ and $R^5$ are as defined, $R^6$ is $C_{1-6}$ alkyl, Y is oxygen or sulphur, X is halogen or $C_{1-6}$ alkoxy and Z is a salting ion;

d) converting a product into a pharmaceutically acceptable salt or ester.

9 A compound of formula (X):

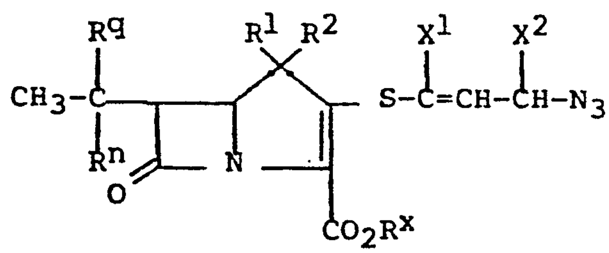

(X)

wherein $R^1$, $R^2$, and $R^n$ are as defined in claim 1; $R^q$ and $R^x$ are as defined in claim 8.

10 A compound of formula (XI):

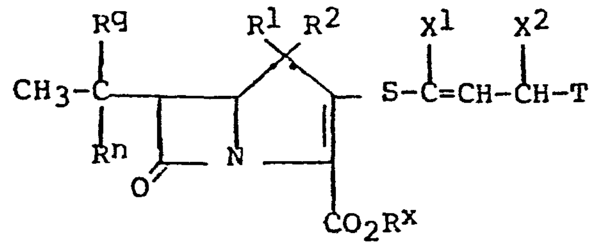

(XI)

wherein $R^1$, $R^2$ and $R^n$ are as defined in claim 1; $R^q$ and $R^x$ are as defined in claim 8 and T represents a readily displaceable group.

11 A compound of formula (XII):

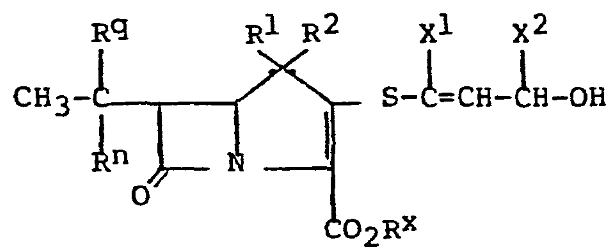

(XII)

wherein $R^1$, $R^2$, $R^n$ and $X^1$ and $X^2$ are as defined in claim 1; $R^2$ and $R^x$ are as defined in claim 8.

12 A compound of formula (XV):

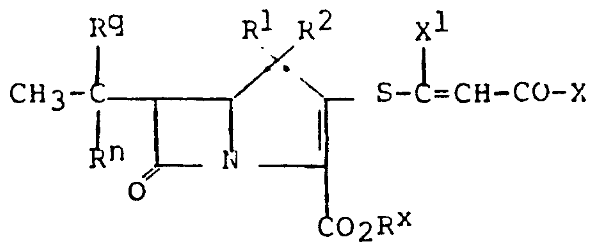

(XV)

wherein $R^1$, $R^2$, $R^n$ and $X^1$ are as defined in claim 1; $R^q$ and $R^x$ are as defined in claim 8 and X represents $X^2$ as defined in claim 1 or $OR^y$ where $CO_2R^y$ represents an ester group; $R^y$ is a hydrocarbon radical.

13 A pharmaceutical composition which comprises a compound of formula (I) and a pharmaceutically acceptable carrier.

14 A compound as claimed in any one of claims 1 to 7 for use in the treatment of antibacterial infections.

0105658

European Patent Office

# EUROPEAN SEARCH REPORT

Application number

EP 83 30 5512

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 001 628 (MERCK)<br>* Pages 73,75, compounds 1,23; claims *<br>----- | 1-6,8, 13 | C 07 D 487/04<br>A 61 K 31/40 //<br>C 07 F 7/18<br>C 07 C 149/18<br>C 07 C 149/24<br>(C 07 D 487/04<br>C 07 D 209/00<br>C 07 D 205/00 ) |
| | | | TECHNICAL FIELDS SEARCHED (Int. Cl. 3) |
| | | | C 07 D 487/00<br>A 61 K 31/00 |

The present search report has been drawn up for all claims

| Place of search | Date of completion of the search | Examiner |
|---|---|---|
| THE HAGUE | 29-11-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document